# EUROPEAN PATENT APPLICATION

(11) **EP 3 950 705 A1**
(43) Date of publication of application: **09.02.2022**
(21) Application number: 20190070.1
(22) Date of filing: 07.08.2020
(51) Int. Cl.: C07K 14/165, A61K 39/215

(54) **PEPTIDES AND COMBINATIONS OF PEPTIDES FOR USE IN IMMUNOTHERAPY AGAINST AN INFECTION BY SARS-COV-2 (COVID-19)**

(71) Applicant: Eberhard Karls Universität Tübingen Medizinische Fakultät, 72074 Tübingen (DE)
(72) Inventor: Walz, Juliane, 72074 Tübingen (DE); Nelde, Annika, 72070 Tübingen (DE); Rammensee, Hans-Georg, 72070 Unterjesingen (DE); Bilich, Tatjana, 72072 Tübingen (DE)
(74) Representative: Witte, Weller & Partner Patentanwälte mbB

(57) **Abstract**

The present invention relates to peptides, proteins, nucleic acids and cells for use in immunotherapeutic methods. In particular, the present invention relates to the immunotherapy of an infection by SARS-CoV-2 (COVID-19). The present invention furthermore relates to SARS-CoV-2-associated T-cell peptide epitopes that can for example serve as active pharmaceutical ingredients of vaccine compositions that stimulate anti-SARS-CoV-2 immune responses, or to stimulate T-cells *ex vivo* and transfer them into patients. Peptides bound to molecules of the major histocompatibility complex (MHC), or peptides as such, can also be targets of antibodies, soluble T-cell receptors, and other binding molecules.

## Description

The present invention relates to peptides, proteins, nucleic acids and cells for use in immunotherapeutic methods. In particular, the present invention relates to the immunotherapy of an infection by SARS-CoV-2 (COVID-19). The present invention furthermore relates to SARS-CoV-2-associated T-cell peptide epitopes that can for example serve as active pharmaceutical ingredients of vaccine compositions that stimulate anti-SARS-CoV-2 immune responses, or to stimulate T-cells ex *vivo* and transfer them into patients. Peptides bound to molecules of the major histocompatibility complex (MHC), or peptides as such, can also be targets of antibodies, soluble T-cell receptors, and other binding molecules.

The present invention relates to several novel peptide sequences and their variants that can be used in vaccine compositions for eliciting anti-SARS-CoV-2 immune responses, or as targets for the development of pharmaceutically/immunologically active compounds and cells.

### FIELD OF THE INVENTION

The present invention relates to the field of molecular biology, more particular to the field of molecular immunology.

### BACKGROUND OF THE INVENTION

The novel coronavirus SARS-CoV-2 is responsible for the COVID-19 disease, which especially in elderly, weakened and immunocompromised patients shows severe and fatal courses. In the meantime, SARS-CoV-2 has spread to a worldwide pandemic with yet incalculable health, economic and socio-political consequences. So far, there are no established therapies and a vaccine is not yet available.

Deaths and serious illness are more common in the older population over 60 years of age. Outbreaks in long-term care facilities have been observed in several countries, which pose particular challenges in terms of containment and isolation within the facility, affecting and threatening those most at risk. For older patients with SARS-CoV-2 infection, a high hospitalization rate between 28.6% and 43.5% in the age group 65-74 years and between 30.5% and 58.7% in the age group 75-84 years has been described, with an associated high mortality rate of up to 30%.

There are two promising options for reducing the number of severe COVID-19 cases in elderly and comorbid people in the future. The first option relates to the development of preventive measures (vaccination) that prevent the disease or reduce its progression. The second option relates to a therapeutic intervention in early stages. Both approaches can prevent deterioration in the course of disease, reduce the frequency of hospital admissions and intensive care treatment and thus take the pressure of the health care system.

T-cell immunity plays an essential role in the control of viral infections. CD4⁺ T-helper cells (Th1) are essential for the regulation and maintenance of the immune response and for the production of antiviral cytokines, whereas cytotoxic CD8⁺ T-cells (CTL) are responsible for the elimination of virus-infected cells. The recognition of viral antigens, which are presented as short peptides via the human leukocyte antigen system (HLA), is essential for the activation and function of T-cells. To identify and analyze protective T-cell immune responses against viral infections in the human population, a comprehensive identification and characterization of such viral T-cell epitopes is necessary. This knowledge is not only essential for understanding the host's immune response and the mechanisms of long-term protection in case of virus recurrence, but also a prerequisite for the development of new and more efficient therapeutic and preventive immunotherapy approaches. Besides the generation of virus-specific T-cells ex *vivo* with subsequent transfer into the patient, the possibility of direct vaccination with T-cell epitopes for the induction of a T-cell response directly *in vivo* is of particular importance. Such vaccines can be used prophylactically in patients at high risk for a severe course of SARS-CoV-2 infection to generate immunity against the infection. Furthermore, they can also be used therapeutically to prevent severe courses of disease by accelerating/generating a virus-specific T-cell response and activating *in vivo* virus-specific B-cells supporting antibody production.

The findings and experience with two other zoonotic coronaviruses - SARS-CoV-1 and MERS-CoV - based on the detection of CoV-specific CD8⁺ and long-lasting CD4⁺ memory T-cell responses in convalescents provide evidence that T-cell immunity also plays an important role in the control of coronavirus infections. Channappanavar et al. Virus-specific memory CD8 T-cells provide substantial protection from lethal severe acute respiratory syndrome coronavirus infection. J Virol. 2014;88(19):11034-11044; Channappanavar et al. T-cell-mediated immune response to respiratory coronaviruses. Immunol Res. 2014;59(1-3):118-128; Zhao et al. Airway Memory CD4(+) T-cells Mediate Protective Immunity against Emerging Respiratory Coronaviruses. Immunity. 2016;44(6):1379-1391; Zhao et al. T-cell responses are required for protection from clinical disease and for virus clearance in severe acute respiratory syndrome coronavirus-infected mice. J Virol. 2010;84(18):9318-9325. This is even more important since studies on humoral immunity to SARS-CoV-1 provided evidence that antibody responses are short-lived and can even cause or aggravate virus-associated lung pathology. Liu et al. Anti-spike IgG causes severe acute lung injury by skewing macrophage responses during acute SARS-CoV infection. JCI Insight. 2019;4(4); Tang et al. Lack of peripheral memory B cell responses in recovered patients with severe acute respiratory syndrome: a six-year follow-up study. J Immunol. 2011;186(12):7264-7268. For CD8⁺ and Th1 CD4⁺ T-cells in contrast a crucial role in viral clearance and protection against the deadly SARS-CoV-1 infection was reported especially in terms of reported lung pathology.

Numerous CD4⁺ and CD8⁺ T-cell epitopes have been described for SARS-CoV-1 and MERS-CoV, which, due to the sequence homology of the two coronaviruses, suggest potential cross-reactivity and could also be potential T-cell epitopes for the new SARS-CoV-2 virus; Liu et al. T-cell immunity of SARS-CoV: Implications for vaccine development against MERS-CoV. Antiviral Res. 2017;137:82-92.

With regard to SARS-CoV-2, two very recent studies described CD4⁺ and CD8⁺ T-cell responses against viral peptide pools in donors that had recovered from COVID-19 as well as individuals not exposed to SARS-CoV-2, indicative of potential T-cell cross-reactivity; see Grifoni et al. Targets of T-cell Responses to SARS-CoV-2 Coronavirus in Humans with COVID-19 Disease and Unexposed Individuals. Cell. 2020; Braun et al. Presence of SARS-CoV-2 reactive T-cells in COVID-19 patients and healthy donors. medRxiv. 2020:2020.2004.2017.20061440.

In own preliminary work, the inventors defined SARS-CoV-2-specific and cross-reactive CD4⁺ and CD8⁺ T-cell epitopes in a large collection of SARS-CoV-2 convalescents as well as non-exposed individuals and confirmed their relevance for immunity and the course of COVID-19 disease; see Nelde et al. SARS-CoV-2 T-cell epitopes define heterologous and COVID-19-induced T-cell recognition. In: Preprint, ed. Research Square 2020. These SARS-CoV-2 T-cell epitopes show high recognition frequencies in convalescents from SARS-CoV-2 infection, suggesting their important role in the natural course and immune control of COVID-19.

In non-published European patent application no. 20 169 047 various SARS-CoV-2-specific peptide sequences are described which are proposed as active ingredients of a vaccination preparation against COVID-19.

Currently, several peptide-based vaccines, for viruses such as EBV, HBV, foot and mouth disease, swine fever, anthrax, malaria, human immunodeficiency virus and influenza virus, are evaluated in clinical trials.

However, so far the SARS-CoV-2-specific peptide sequences described so far have not been proven in practice and especially not in clinical use. In particular, no peptide-based vaccine effective against an infection by SARS-CoV-2 is available.

It is, therefore, an object underlying the invention to provide SARS-CoV-2-derived T-cell epitopes which can be used to develop medicaments and therapeutic methods for the prophylaxis and treatment of an infection by SARS-CoV-2 (COVID-19), which may also allow a better understanding of the biology of SARS-CoV-2 and its transmission.

The present invention satisfies these and other needs.

### SUMMARY OF THE INVENTION

The present invention provides a peptide comprising an amino acid sequence selected from the group consisting of SEQ ID NO: 1 to SEQ ID NO: 10 or consisting of SEQ ID NO: 1, 2, 3, 4, 7, 8, 9, and 10, and variant sequences thereof which are at least 88% homologous to SEQ ID NO: 1 to SEQ ID NO: 10 or SEQ ID NO: 1, 2, 3, 4, 7, 8, 9, and 10, and wherein said variant binds to molecule(s) of the major histocompatibility complex (MHC) and/or induces T-cells cross-reacting with said variant peptide; and a pharmaceutical acceptable salt thereof, wherein said peptide is not a full-length polypeptide.

The inventors were able to predict ten promiscuous HLA-DR peptides from different proteins or open reading frames (ORFs) of the SARS-CoV-2 virus, respectively. Using the algorithm SYFPEITHI peptide sequences were selected that show a high binding score for several HLA-DR allotypes and can therefore be widely used in the population.

Furthermore, especially those HLA-DR peptides were selected by the inventors that contain embedded HLA class I sequences in order to induce CD4⁺ T-cell responses as well as CD8⁺ T-cell responses.

Furthermore, for peptides from virus surface proteins, only sequences were selected that do not represent antibody epitopes, since they are not accessible to antibodies due to the predicted 3D structure of the protein. This should prevent the formation of antibodies against the vaccinated peptides, which could possibly lead to a deterioration of the lung pathology.

Immunogenicity was proven for all HLA-DR peptides included in a peptide cocktail in a large cohort of SARS-CoV-2 convalescent donors proving the significant role of these T-cell epitopes in the natural course of SARS-CoV-2 infection. Furthermore, two peptide sequences were applied in a vaccination approach in a single healthy volunteer, inducing strong CD4⁺ T-cell responses.

The SARS-CoV-2-derived HLA-DR peptides according to the invention activate CD4⁺Th1 cells which directly contribute to virus clearance and deliver strong T-helper signals to the CD8⁺ T-cells primed by embedded HLA class I sequences in a vaccine or during natural infection. Furthermore, in terms of a SARS-CoV-2 infection these SARS-CoV-2 specific CD4⁺Th1 cells should vigorously activate virus antigen-experienced B cells. The resulting enhanced activity could lead to more rapid virus clearance and prevention of severe course of COVID-19.

The term "peptide" is used herein to designate a series of amino acid residues, connected one to the other typically by peptide bonds between the alpha-amino and carbonyl groups of the adjacent amino acids. The peptides are preferably between 7 and 20 amino acids in length, further preferably between 8 and 19, and highly preferably of 15 amino acids in length, but can be as long as 3, 4, 5, 6, 7, 8, 9, 10, 11, 12, 13, 14, 15, 16, 17, 18, 19, 20 or longer.

Furthermore, the term "peptide" shall include salts of a series of amino acid residues, connected one to the other typically by peptide bonds between the alpha-amino and carbonyl groups of the adjacent amino acids. Preferably, the salts are pharmaceutical acceptable salts of the peptides, such as, for example, the chloride or acetate (trifluoroacetate) salts. It has to be noted that the salts of the peptides according to the present invention differ substantially from the peptides in their state(s) *in vivo,* as the peptides are not salts *in vivo.*

The term "peptide" shall also include "oligopeptide". The term "oligopeptide" is used herein to designate a series of amino acid residues, connected one to the other typically by peptide bonds between the alpha-amino and carbonyl groups of the adjacent amino acids. The length of the oligopeptide is not critical to the invention, as long as the correct epitope or epitopes are maintained therein. The oligopeptides are typically less than about 30 amino acid residues in length, and greater than about 10 or 15 amino acids in length.

The term "peptide" shall also include "polypeptide". The term "polypeptide" designates a series of amino acid residues, connected one to the other typically by peptide bonds between the alpha-amino and carbonyl groups of the adjacent amino acids. The length of the polypeptide is not critical to the invention as long as the correct epitopes are maintained. In contrast to the terms peptide or oligopeptide, the term polypeptide is meant to refer to molecules containing more than about 30 amino acid residues.

By a "variant" of the given amino acid sequence the inventors mean that the side chains of, for example, one or two of the amino acid residues are altered (for example by replacing them with the side chain of another naturally occurring amino acid residue or some other side chain) such that the peptide is still able to bind to an MHC molecule in substantially the same way as a peptide consisting of the given amino acid sequence of SEQ ID NO: 1 to SEQ ID NO: 10 or SEQ ID NO: 1, 2, 3, 4, 7, 8, 9, and 10. For example, a peptide may be modified so that it at least maintains, if not improves, the ability to interact with and bind to the binding groove of a suitable MHC molecule, such as HLA-A*02, and in that way, it at least maintains, if not improves, the ability to bind to the TCR of activated T-cells.

A person skilled in the art will be able to assess whether T-cells induced by a variant of a specific peptide will be able to cross-react with the peptide itself (Appay et al., 2006; Colombetti et al., 2006; Fong et al., 2001; Zaremba et al., 1997).

These T-cells can subsequently cross-react with cells and kill cells that express a polypeptide that contains the natural amino acid sequence of the cognate peptide as defined in the aspects of the invention. As can be derived from the scientific literature and databases (Rammensee et al., 1999 *I.c*.; Godkin et al., 1997), certain positions of HLA binding peptides are typically anchor residues forming a core sequence fitting to the binding motif of the HLA receptor, which is defined by polar, electrophysical, hydrophobic and spatial properties of the polypeptide chains constituting the binding groove. Thus, one skilled in the art would be able to modify the amino acid sequences set forth in SEQ ID NO: 1 to SEQ ID NO: 10 or SEQ ID NO: 1, 2, 3, 4, 7, 8, 9, and 10, by maintaining the known anchor residues, and would be able to determine whether such variants maintain the ability to bind MHC class I or II molecules. The variants of the present invention retain the ability to bind to the TCR of activated T-cells, which can subsequently cross-react with and kill cells that express a polypeptide containing the natural amino acid sequence of the cognate peptide as defined in the aspects of the invention.

In the present invention, the term "homologous" refers to the degree of identity between sequences of two amino acid sequences, i.e. peptide or polypeptide sequences. The aforementioned "homology" is determined by comparing two sequences aligned under optimal conditions over the sequences to be compared. Such a sequence homology can be calculated by creating an alignment using, for example, the ClustalW algorithm. Commonly available sequence analysis software, more specifically, Vector NTI, GENETYX or other tools are provided by public databases.

"Percent identity" or "percent identical" in turn, when referring to a sequence, means that a sequence is compared to a claimed or described sequence after alignment of the sequence to be compared (the "Compared Sequence") with the described or claimed sequence (the "Reference Sequence"). The percent identity is then determined according to the following formula: percent identity = 100 [1 -(C/R)]
wherein C is the number of differences between the Reference Sequence and the Compared Sequence over the length of alignment between the Reference Sequence and the Compared Sequence, wherein
   (i) each base or amino acid in the Reference Sequence that does not have a corresponding aligned base or amino acid in the Compared Sequence and
   (ii) each gap in the Reference Sequence and
   (iii) each aligned base or amino acid in the Reference Sequence that is different from an aligned base or amino acid in the Compared Sequence, constitutes a difference and
   (iiii) the alignment has to start at position 1 of the aligned sequences;
and R is the number of bases or amino acids in the Reference Sequence over the length of the alignment with the Compared Sequence with any gap created in the Reference Sequence also being counted as a base or amino acid.

If an alignment exists between the Compared Sequence and the Reference Sequence for which the percent identity as calculated above is about equal to or greater than a specified minimum Percent Identity then the Compared Sequence has the specified minimum percent identity to the Reference Sequence even though alignments may exist in which the herein above calculated percent identity is less than the specified percent identity.

According to the invention "full-length polypeptide" refers to the source proteins from which the peptides are derived, e.g. SARS-CoV-2 encoded proteins, such as the 7096 amino acid (aa) long ORF1ab polyprotein (replicase complex), the 1273 aa long surface glycoprotein (S for "spikes"; ORF2), the 75 aa long envelope protein (E for "envelope"; ORF 4), the 222 aa long membrane glycoprotein (M for "membrane; ORF5), the 419 aa long nucleocapsid phosphoprotein (N for "nucleocapsid"; ORF9) and another five proteins (ORF3a, ORF6, ORF7a, ORF8 and ORF10).

The problem underlying the invention is herewith completely solved.

In an embodiment of the invention said peptide has the ability to bind to an MHC class-I or -II molecule, and wherein said peptide, when bound to said MHC, is capable of being recognized by CD4 and/or CD8 T-cells.

This measure has the advantage that the capability of the peptide according to the invention to induce an immune response, in particular a T-cell response, is ensured.

In another embodiment of the invention the amino acid sequence thereof comprises a continuous stretch of amino acids according to any one of SEQ ID NO: 1 to SEQ ID NO: 10 or SEQ ID NO: 1, 2, 3, 4, 7, 8, 9, and 10.

This measure has the advantage that the peptide according to the invention comprises all amino acids which are predicted as being involved in the induction of an immune response. The therapeutic efficacy is herewith further improved. A "continuous stretch" in this context means that the peptide according to the invention comprises any of the amino acid sequence of SEQ ID NO: 1 to SEQ ID NO: 10 or SEQ ID NO: 1, 2, 3, 4, 7, 8, 9, and 10, without any interruption by or insertion of other components or amino acids, e.g. AS₁-AS₂-AS₃-AS₄-AS₅-AS₆-AS₇-AS₈-AS₉-AS₁₀, or AS₁-AS₂-AS₃-AS₄-AS₇-AS₈-AS₉-AS₁₀, where 'AS' stands for amino acid and '-' represents a peptide binding.

Another subject-matter of the present invention relates to an antibody, in particular a soluble or membrane-bound antibody, preferably a monoclonal antibody or fragment thereof, which specifically recognizes the peptide or variant thereof according to the invention, preferably when bound to an MHC molecule.

The term "antibody" or "antibodies" is used herein in a broad sense and includes both polyclonal and monoclonal antibodies. In addition to intact or "full" immunoglobulin molecules, also included in the term "antibodies" are fragments (e.g. CDRs, Fv, Fab and Fc fragments) or polymers of those immunoglobulin molecules and humanized versions of immunoglobulin molecules, as long as they exhibit any of the desired properties, i.e. specifically recognize the peptide or variant thereof according to the invention. Whenever possible, the antibodies of the invention may be purchased from commercial sources. The antibodies of the invention may also be generated using well-known methods.

The features, characteristics, advantages and embodiments disclosed for the peptide according to the invention apply to the antibody and fragment thereof correspondingly.

Another subject-matter of the invention relates to a T-cell receptor, preferably soluble or membrane-bound, or a fragment thereof, that is reactive with an HLA ligand, wherein said ligand is the peptide or variant thereof according to the invention, preferably when bound to an MHC molecule.

The term "T-cell receptor" (abbreviated TCR) according to the invention refers to a heterodimeric molecule comprising an alpha polypeptide chain (alpha chain) and a beta polypeptide chain (beta chain), wherein the heterodimeric receptor is capable of binding to a peptide antigen presented by an HLA molecule. The term also includes so-called gamma/delta TCRs.

The features, characteristics, advantages and embodiments disclosed for the peptide and antibody or fragment thereof according to the invention apply to the T-cell receptor correspondingly.

A still further subject-matter according to the invention relates to a nucleic acid, encoding for a peptide or variant thereof according to the invention, an antibody or fragment thereof according to the invention, a T-cell receptor or fragment thereof according to the invention, optionally linked to a heterologous promoter sequence, or an expression vector expressing said nucleic acid.

The nucleic acid coding for a particular peptide, oligopeptide, or polypeptide may be naturally occurring or they may be synthetically constructed. The nucleic acid (for example a polynucleotide) may be, for example, DNA, cDNA, PNA, RNA or combinations thereof, either single- and/or double- stranded, or native or stabilized forms of polynucleotides, such as, for example, polynucleotides with a phosphorothioate backbone and it may or may not contain introns so long as it codes for the peptide. Of course, only peptides that contain naturally occurring amino acid residues joined by naturally occurring peptide bonds are encodable by a polynucleotide. A still further aspect of the invention provides an expression vector capable of expressing a polypeptide according to the invention. As used herein the term "nucleic acid coding for (or encoding) a peptide" refers to a nucleotide sequence coding for the peptide including artificial (man-made) start and stop codons compatible for the biological system the sequence is to be expressed by, for example, a dendritic cell or another cell system useful for the production of TCRs. The term "promoter" means a region of DNA involved in binding of RNA polymerase to initiate transcription.

The features, characteristics, advantages and embodiments disclosed for the peptide according to the invention apply to the nucleic acid and expression vector correspondingly.

Another subject-matter of the invention relates to a recombinant host cell comprising the peptide according to the invention, the antibody or fragment thereof according to the invention, the T-cell receptor or fragment thereof according to the invention or the nucleic acid or the expression vector according to the invention, wherein said host cell preferably is selected from an antigen presenting cell, such as a dendritic cell, a T-cell or an NK cell.

The features, characteristics, advantages and embodiments disclosed for the peptide according to the invention apply to the host cell correspondingly.

A still further subject-matter of the invention relates to an *in vitro* method for producing activated T lymphocytes, the method comprising contacting *in vitro* T-cells with antigen loaded human class I or II MHC molecules expressed on the surface of a suitable antigen-presenting cell or an artificial construct mimicking an antigen-presenting cell for a period of time sufficient to activate said T-cells in an antigen specific manner, wherein said antigen is a peptide according to the invention.

The activated T-cells that are directed against the peptides of the invention are useful in therapy. Thus, a further aspect of the invention provides activated T-cells obtainable by the foregoing methods of the invention.

Activated T-cells, which are produced by the above method, will selectively recognize a cell that aberrantly expresses a polypeptide that comprises an amino acid sequence of SEQ ID NO: 1 to SEQ ID NO: 10 or SEQ ID NO: 1, 2, 3, 4, 7, 8, 9, and 10.

Another subject-matter according to the invention relates to a pharmaceutical composition comprising at least one active ingredient selected from the group consisting of the peptide according to the invention, the antibody or fragment thereof according to the invention, the T-cell receptor or fragment thereof according to the invention, the nucleic acid or the expression vector according to the invention, the recombinant host cell according to the invention, or the activated T lymphocyte according to the invention, or a conjugated or labelled active ingredient, and a pharmaceutically acceptable carrier, and optionally, pharmaceutically acceptable excipients and/or stabilizers.

A "pharmaceutical composition" is a composition suitable for administration to a human being in a medical setting. Preferably, a pharmaceutical composition is sterile and produced according to GMP guidelines.

The pharmaceutical compositions comprise the peptides either in the free form or in the form of a pharmaceutically acceptable salt (see also above). As used herein, "a pharmaceutically acceptable salt" refers to a derivative of the disclosed peptides wherein the peptide is modified by making acid or base salts of the agent. For example, acid salts are prepared from the free base (typically wherein the neutral form of the drug has a neutral -NH₂ group) involving reaction with a suitable acid. Suitable acids for preparing acid salts include both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methane sulfonic acid, ethane sulfonic acid, p-toluenesulfonic acid, salicylic acid, and the like, as well as inorganic acids, e.g., hydrochloric acid, hydrobromic acid, sulfuric acid, nitric acid phosphoric acid and the like. Conversely, preparation of basic salts of acid moieties which may be present on a peptide are prepared using a pharmaceutically acceptable base such as sodium hydroxide, potassium hydroxide, ammonium hydroxide, calcium hydroxide, trimethylamine or the like.

Preferably, the pharmaceutical composition of the present invention is an immunotherapeutic such as a vaccine. It may be administered directly into the patient, preferably subcutaneously (s.c.), e.g. in the abdomen, or otherwise systemically, i.d., i.m., i.p. and i.v., or applied *ex vivo* to cells derived from the patient or a human cell line which are subsequently administered to the patient, or used *in vitro* to select a subpopulation of immune cells derived from the patient, which are then re-administered to the patient. If the nucleic acid is administered to cells *in vitro,* it may be useful for the cells to be transfected so as to co-express immune-stimulating cytokines, such as interleukin-2. The peptide may be substantially pure, or combined with an immune-stimulating adjuvant or used in combination with immune-stimulatory cytokines, or be administered with a suitable delivery system, for example liposomes. The peptide may also be conjugated to a suitable carrier such as keyhole limpet haemocyanin (KLH) or mannan (see WO 95/18145 and Longenecker et al., 1993). The peptide according to the invention may also be tagged, may be a fusion protein, or may be a hybrid molecule. The peptides whose sequence is given in the present invention are expected to stimulate CD4 or CD8 T-cells. However, stimulation of CD8 T-cells is more efficient in the presence of help provided by CD4 T-helper cells. Thus, for MHC Class I epitopes that stimulate CD8 T-cells the fusion partner or sections of a hybrid molecule suitably provide epitopes which stimulate CD4-positive T-cells. CD4- and CD8-stimulating epitopes are well known in the art and include those identified in the present invention.

In one aspect, the vaccine comprises at least one peptide having the amino acid sequence set forth SEQ ID NO: 1 to SEQ ID NO: 10 or SEQ ID NO: 1, 2, 3, 4, 7, 8, 9, and 10, and at least one additional peptide, preferably two to 50, more preferably two to 25, even more preferably two to 20 and most preferably two, three, four, five, six, seven, eight, nine, ten, eleven, twelve, thirteen, fourteen, fifteen, sixteen, seventeen or eighteen peptides. The peptide(s) may be derived from one or more specific TAAs and may bind to MHC class I molecules.

In an embodiment of the pharmaceutical composition according to the invention it comprises at least 5, preferably at least 6, further preferably at least 7, further preferably at least 8, further preferably at least 9, and further preferably at least 10 different peptides, each peptide comprising an amino acid sequence selected from the group consisting of SEQ ID NO: 1 to SEQ ID NO: 10 or SEQ ID NO: 1, 2, 3, 4, 7, 8, 9, and 10, and variant sequences thereof which are at least 88% homologous to SEQ ID NO: 1 to SEQ ID NO: 10 or SEQ ID NO: 1, 2, 3, 4, 7, 8, 9, and 10, and wherein said variant binds to molecule(s) of the major histocompatibility complex (MHC) and/or induces T-cells cross-reacting with said variant peptide; and a pharmaceutical acceptable salt thereof, wherein said peptide is not a full-length polypeptide.

The inventors have realized that a pharmaceutical composition, such as a vaccine, has a good immunogenic effect if at least 5 peptides or amino acid sequences out of the SEQ ID NOS: 1 to 10 or out of SEQ ID NO: 1, 2, 3, 4, 7, 8, 9, and 10 are contained therein. When using 8 or 10 peptides or amino acid sequences 91.7% of the human world population may be covered.

In a preferred embodiment of the invention the pharmaceutical composition, preferably being a vaccine, comprising peptides characterized by the following amino acid sequences SEQ ID NO: 1-10:
KDGIIWVATEGALNT (SEQ ID NO: 1)
RWYFYYLGTGPEAGL (SEQ ID NO: 2)
ASWFTALTQHGKEDL (SEQ ID NO: 3)
LLLLDRLNQLESKMS (SEQ ID NO: 4)
ASAFFGMSRIGMEVT (SEQ ID NO: 5)
VADYSVLYNSASFST (SEQ ID NO: 6)
ITRFQTLLALHRSYL (SEQ ID NO: 7)
LSYYKLGASQRVAGD (SEQ ID NO: 8)
FYVYSRVKNLNSSRV (SEQ ID NO: 9)
SKWYIRVGARKSAPL (SEQ ID NO: 10).

Such vaccine is referred to by the inventors as 'CoVac-1 vaccine' and has turned out as providing particularly effective protection.

In yet another preferred embodiment of the invention the pharmaceutical composition, preferably being a vaccine, comprising peptides characterized by the following amino acid sequences SEQ ID NO: 1, 2, 3, 4, 7, 8, 9, and 10:
KDGIIWVATEGALNT (SEQ ID NO: 1)
RWYFYYLGTGPEAGL (SEQ ID NO: 2)
ASWFTALTQHGKEDL (SEQ ID NO: 3)
LLLLDRLNQLESKMS (SEQ ID NO: 4)
ITRFQTLLALHRSYL (SEQ ID NO: 7)
LSYYKLGASQRVAGD (SEQ ID NO: 8)
FYVYSRVKNLNSSRV (SEQ ID NO: 9)
SKWYIRVGARKSAPL (SEQ ID NO: 10).

The inventors have found out that the omission of peptides comprising the sequences SEQ ID NO: 5 and 6 results in a significant increase of the solubility of the peptide cocktail.

In a preferred embodiment the pharmaceutical composition additionally comprises an adjuvant.

While peptide vaccines are an attractive strategy to induce highly targeted immune responses, consequently avoiding allergenic and/or reactogenic sequences, they are often weakly immunogenic and require particulate carriers for delivery and adjuvating. Therefore, beside the selection of optimal peptide targets, the usage of a suitable adjuvant, which is able to induce strong and long-lasting immune responses, is preferred. According to the invention, any kind of suitable immunogenic adjuvant comes into consideration, such as water-in-oil emulsions, aluminium salts, Freund's complete adjuvant, squalene, QS21, lipid A, etc.

In an embodiment of the pharmaceutical composition the adjuvant is selected from the group consisting of TLR1/2 ligand, TLR1/2 ligand XS15, TLR1/2 ligand Pam3Cys, TLR9 ligand, TLR9 ligand CpG, Montanide ISA 51 VG, and combinations thereof.

This measure has the advantage that especially effective adjuvants are provided. Among the most effective peptide vaccination methods tested in humans is the subcutaneous injection of peptides emulsified in Montanide ISA 51 VG, a water-in-oil-emulsion, combined with the TLR9 ligand CpG. The novel TLR1/2 ligand XS15 emulsified in Montanide ISA 51 VG can also be employed as adjuvant. XS15 is a water-soluble derivative of the TLR1/2 ligand Pam3Cys inducing a strong CD8⁺ and Th1 CD4⁺ T-cell response against free short peptides in Montanide ISA 51 VG after a single s.c. injection in healthy volunteers as well as in patients.

The appropriate dosage regimen of the pharmaceutical preparation according to the invention will be established by the skilled person by dose finding studies under consideration of various factors such as the age, sex and medical history of the patient, the severity of the expected disease, etc. Typically, peptide doses ranging from 10 to 5,000 µg per vaccination can be used. Preferably, peptide doses ranging from 300-500 µg per vaccination are used which induce strong immune responses. In an embodiment of the invention, the preferred dose for a peptide is in the range between 100 to 500 µg with a strong preference to the upper range. The dose of -240 µg per peptide per dose for a vaccine according to the invention is preferred in another embodiment of the pharmaceutical composition. With this dose induction of strong T-cell response for the peptides included in the vaccine in combination with XS15 emulsified in Montanide ISA 51 VG could be shown. In an embodiment of the invention the pharmaceutical composition is provided in a dosage form where the peptide is contained in the amount as indicated in this paragraph. This measure has the advantage that a ready-to-use vaccine is provided.

Another subject-matter of the invention relates to the peptide according to the invention, the antibody or fragment thereof according to the invention, the T-cell receptor or fragment thereof according to the invention, the nucleic acid or the expression vector according to the invention, the recombinant host cell according to the invention, or the activated T lymphocyte according to the invention for use in medicine, preferably for use against an infection by SARS-CoV-2 (COVID-19), such as a vaccine.

A still further subject-matter according to the invention relates to a kit comprising:
(a) a container comprising a pharmaceutical composition containing the peptide(s) or the variant according to the invention, the antibody or fragment thereof according to the invention, the T-cell receptor or fragment thereof according to the invention, the nucleic acid or the expression vector according to the invention, the recombinant host cell according to the invention, or the activated T lymphocyte according to the invention, in solution or in lyophilized form;
(b) optionally, a second container containing a diluent or reconstituting solution for the lyophilized formulation;
(c) optionally, at least one more peptide selected from the group consisting of SEQ ID NO: 1 to SEQ ID NO: 10 or SEQ ID NO: 1, 2, 3, 4, 7, 8, 9, and 10, and
(d) optionally, instructions for (i) use of the solution or (ii) reconstitution and/or use of the lyophilized formulation.

The kit may further comprise one or more of (iii) a buffer, (iv) a diluent, (v) a filter, (vi) a needle, or (v) a syringe. The container is preferably a bottle, a vial, a syringe or test tube; and it may be a multi-use container. The pharmaceutical composition is preferably lyophilized.

Kits of the present invention preferably comprise a lyophilized formulation of the present invention in a suitable container and instructions for its reconstitution and/or use. Suitable containers include, for example, bottles, vials (e.g. dual chamber vials), syringes (such as dual chamber syringes) and test tubes. The container may be formed from a variety of materials such as glass or plastic. Preferably the kit and/or container contain/s instructions on or associated with the container that indicates directions for reconstitution and/or use. For example, the label may indicate that the lyophilized formulation is to be reconstituted to peptide concentrations as described above. The label may further indicate that the formulation is useful or intended for subcutaneous administration.

It is to be understood that the before-mentioned features and those to be mentioned in the following cannot only be used in the combination indicated in the respective case, but also in other combinations or in an isolated manner without departing from the scope of the invention.

### EMBODIMENTS

The invention is now further explained by means of embodiments resulting in additional features, characteristics and advantages of the invention. The embodiments are of pure illustrative nature and do not limit the scope or range of the invention. The features mentioned in the specific embodiments are features of the invention and may be seen as general features which are not applicable in the specific embodiment but also in an isolated manner in the context of any embodiment of the invention.

The invention is now further described and explained in further detail by referring to the following non-limiting examples and figures:

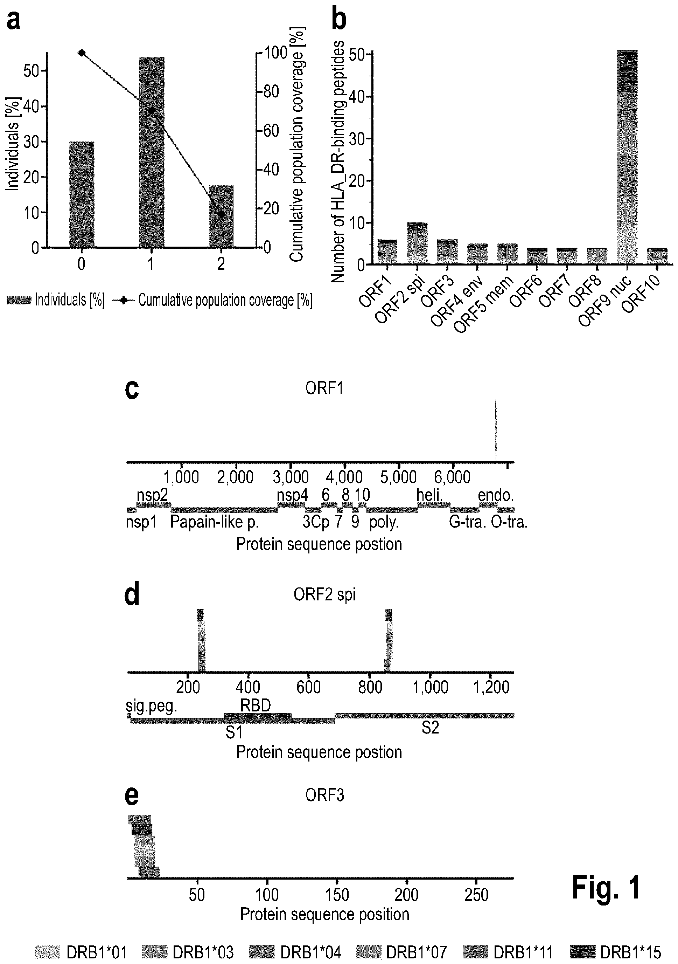

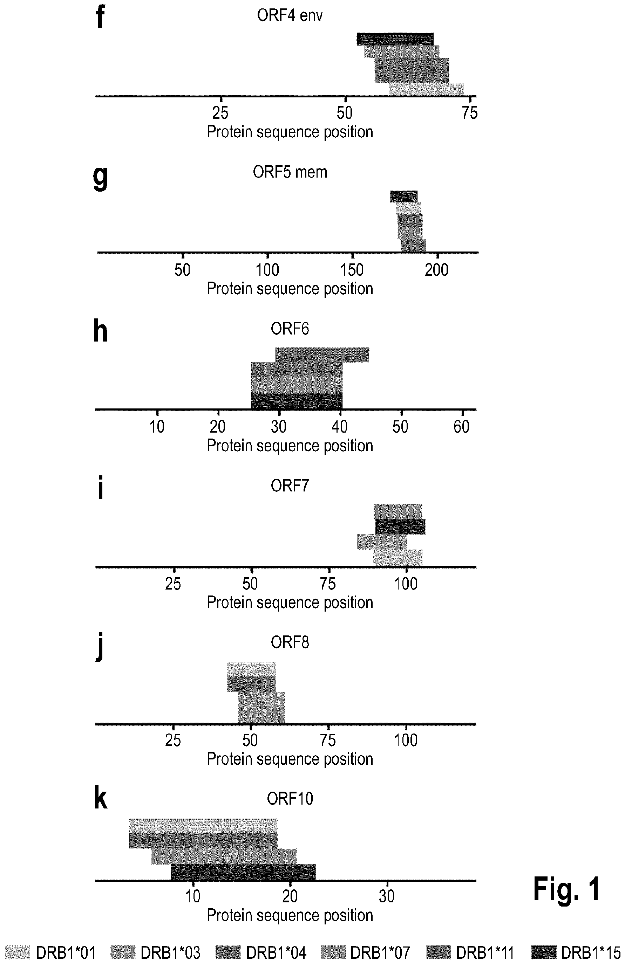

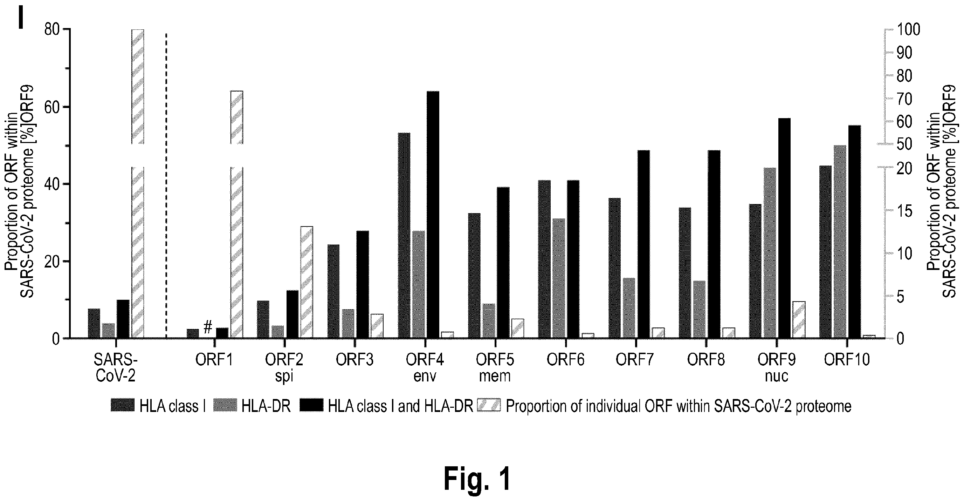

- Fig. 1:: *Prediction of SARS-CoV-2-derived CD4*⁺ *T-cell epitopes.* (A) HLA-DR population coverage reached with the inventors' selection of allotypes compared to the world population. The frequencies of individuals within the world population carrying up to six HLA allotypes (x-axis) are indicated as grey bars on the left y-axis. The cumulative percentage of population coverage is depicted as black dots on the right y-axis. (B) Distribution of HLA-restricted peptides derived from the distinct ORFs to the different HLA-DR allotypes. (C-K) Distribution of HLA-DR-binding peptides within the different ORF proteins. Each color represents a distinct HLA class I allotype. (L) Protein coverage of the predicted HLA-DR peptides for the different ORF proteins. The dashed bar indicated the percentage a distinct ORF covers within the complete SARS-CoV-2 proteome.

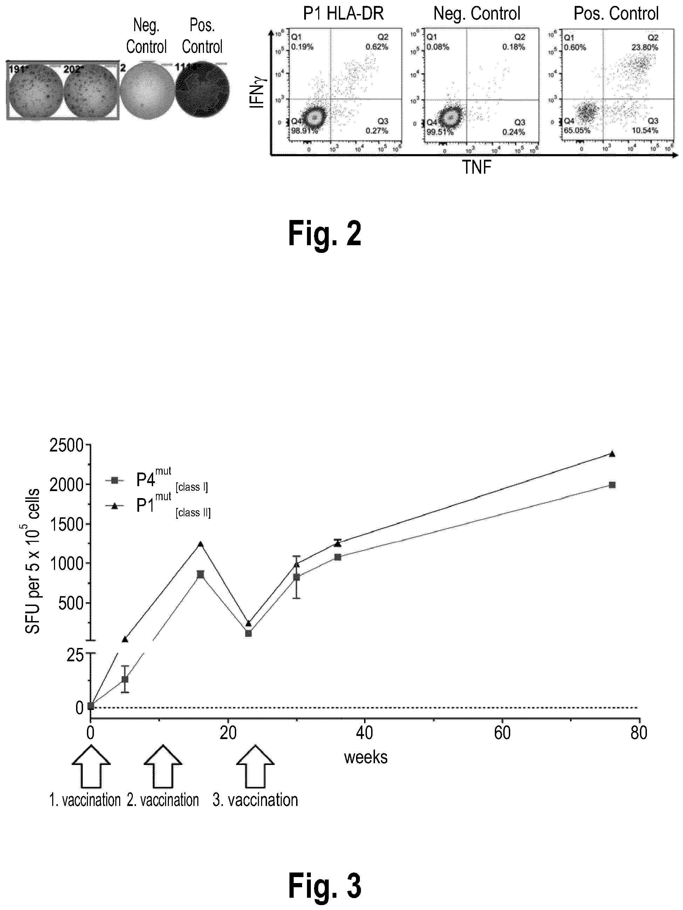

- Fig. 2:: *CD4*⁺ *T-cell responses against CoVac-1 peptides in patients after recovery from SARS-CoV-2 infection.* (A) Example of ELISPOT assays after 12 days of T-cell amplification using KDGIIWVATEGALNT (P01; SEQ ID NO: 1), RWYFYYLGTGPEAGL (P03; SEQ ID NO: 2), LLLLDRLNQLESKMS (P07; SEQ ID NO: 4) in PBMC of patients recovered from SARS-CoV-2 infection HIV DR peptide served as negative control. (B) Intracellular cytokine staining (ICS) of CD4⁺ T-cells using the CoVac-1 peptides KDGIIWVATEGALNT (P01; SEQ ID NO: 1), ASWFTALTQHGKEDL (P04; SEQ ID NO: 3), LLLLDRLNQLESKMS (P07; SEQ ID NO: 4) Graphs show single, viable CD4⁺ cells stained for IFN, TNF and CD107 analyzed by flow cytometry.
- Fig. 3: *Results of IFNy ELISpot-based immunomonitoring in a colon carcinoma patient.* Immunomonitoring was performed before and after s.c. administration of a personalized peptide vaccine containing XS15 emulgated in Montanide. The functionality (IFNγ production) of peptide-specific CD4⁺ and CD8⁺ T-cells for two peptides contained in the administered vaccine is shown. A total of three vaccinations were performed.

### 1. Non-clinical studies

### 1.1 Overview

In the following sections, the selection of the SARS-CoV-2-derived peptides as basis for an embodiment of the vaccine according to the invention (in the following: CoVac-1 vaccine) is explained. Furthermore, all preclinical data for the novel TLR1/2 agonist XS15 included as a lipopeptide in the peptide cocktail for the CoVac-1 drug product (DP) will be described. The predictive value of animal models for the non-clinical testing of CoVac-1 vaccines is limited, as the interaction between HLA molecules, peptides and T-cells is highly species and allele-specific. Therefore, the non-clinical section describing the SARS-CoV-2-derived peptides for the CoVac-1 DP contains extensive *in vitro* and *ex vivo* data.

### 1.2 SARS-CoV-2-derived HLA-DR peptide selection

### Rational for the selection of HLA-class II-derived peptides

Multiple studies in animal models have clearly demonstrated the requirement of CD4⁺ T-cell help for the generation of protective antibody responses (for example, influenza, malaria, vaccinia). Recent studies have also demonstrated that the role of CD4⁺ T-cells in the immune response to viral infections is not limited to help for antibody production; CD4⁺ T-cells are also required to generate optimal CD8⁺ T-cell responses. Moreover, CD4⁺ T-cells additionally can act as effector cells by the secretion of cytokines and direct killing of infected cells. HLA class II antigens specifically activate CD4⁺ helper T-cells, therefore a vaccine based on SARS-CoV-2-derived HLA class II peptides will enable a potent cellular and humoral immune response to SARS-CoV-2 preventing severe courses of COVID-19. Therefore, the CoVac-1 DP was composed of ten SARS-CoV-2 derived HLA-DR peptides.

### Prediction of SARS-CoV-2-derived peptides

For the prediction of potential SARS-CoV-2-derived T-cell epitopes the inventors retrieved the complete highly conserved and annotated representative proteome sequence of SARS-CoV-2 isolate Wuhan-Hu-1 containing ten different open reading frames (ORFs); see Wu et al. A new coronavirus associated with human respiratory disease in China. Nature. 2020;579(7798):265-269. The amino acid sequence of this sequence is completely identical to the reference sequence (EPI_ISL_412026) defined by Wang et al. conducting multiple sequence alignments and phylogenetic analyses of 95 full-length genomic sequences; see Wang et al. The establishment of reference sequence for SARS-CoV-2 and variation analysis. Journal of Medical Virology. 2020.

Using the SYFPEITHI algorithm (Rammensee et al. SYFPEITHI: database for MHC ligands and peptide motifs. Immunogenetics. 1999;50(3-4):213-219) the inventors predicted SARS-CoV-2-derived HLA class II peptides for the six most common HLA-DR allotypes (HLA-DRB1*01:01, -DRB1*03:01, DRB1*04:01, -DRB1*07:01,-DRB1*11:01, and -DRB1*15:01) covering more than 70.0% of the world population with at least one allotype (Figure 1). As the other two HLA class II loci (DQ and DP) with additional binding opportunities for CoVac-1 HLA class II peptides have not been included into the analysis, the real promiscuity of the chosen peptides is probably even higher. Binding of the warehouse HLA class II peptides to several different HLA class II molecules (promiscuous binding) is important to ensure that the majority of patients treated with warehouse peptides are able to benefit from a supportive helper T-cell response. For HLA class II predictions all ten ORFs were split into 15 amino acid long peptides, resulting in 9,561 15mer long peptides in total. The 5% top-scoring peptides of each ORF (related to the total length of each ORF, 2% for ORF1) were selected and sorted according to their position within the protein. To allow for a broad applicability the inventors selected clusters of peptide length variants with high prediction scores for several HLA-DR alleles (Figure 1). From each selected cluster one representative peptide was produced as synthetic peptide for immunogenicity analyses, thereby avoiding peptides containing cysteines (Table 1).

**Table 1: SARS-CoV-2-derived peptided included in CoVac-1 vaccines**

| **sequence** | **SEQ ID NO** | **HLA restriction** | **peptide length** | **position** | **protein** | **protein name** | **protein class** |
|---|---|---|---|---|---|---|---|
| KDGIIWVATEGALNT | 1 | DR | 15 | 127-141 | ORF9 | nucleocapsid protein | structural |
| RWYFYYLGTGPEAGL | 2 | DR | 15 | 107-121 | ORF9 | nucleocapsid protein | structural |
| ASWFTALTQHGKEDL | 3 | DR | 15 | 50-64 | ORF9 | nucleocapsid protein | structural |
| LLLLDRLNQLESKMS | 4 | DR | 15 | 221-235 | ORF9 | nucleocapsid protein | structural |
| ASAFFGMSRIGMEVT | 5 | DR | 15 | 311-325 | ORF9 | nucleocapsid protein | structural |
| VADYSVLYNSASFST | 6 | DR | 15 | 362-376 | ORF2 | spike protein | structural |
| ITRFQTLLALHRSYL | 7 | DR | 15 | 235-249 | ORF9 | spike protein | structural |
| LSYYKLGASQRVAGD | 8 | DR | 15 | 176-190 | ORF5 | membrane protein | structural |
| FYVYSRVKNLNSSRV | 9 | DR | 15 | 56-70 | ORF4 | membrane protein | structural |
| SKWYIRVGARKSAPL | 10 | DR | 15 | 43-57 | ORF8 | n.a. | non-structural |

### Immunogenicity and role in the natural course of SARS-CoV-2 infection of SARS-CoV-2-derived peptides

In order to exert anti-viral activity, peptides contained in the warehouse must (i) be processed and presented naturally in the course of SARS-CoV-2 infection and (ii) induce specific immune responses *in vivo.* To assess these points, the inventors screened a cohort of patients recovered from SARS-CoV-2 infection for T-cell responses against the predicted SARS-CoV-2-derived HLA class II peptides. Naturally-induced peptide-specific T-cell responses were assessed using IFNγ ELISPOT and flow cytometry-based intracellular cytokine staining. Those peptides with the highest frequency (up to 95%) of T-cell responses within the natural SARS-CoV-2 infection were selected for the CoVac-1 vaccine (Table 2). Naturally-induced SARS-CoV-2 T-cells detecting CoVac-1 peptides where further characterized by intracellular cytokine staining. Most T-cells were multifunctional CD4⁺ Th1 cells producing TNF and IFNγ as well as the upregulation of the cytotoxicity marker CD107. Furthermore, the inventors screened a large cohort of donors never exposed to SARS-CoV-2 (samples asserted before 6/2019) for memory T-cell responses against the CoVac-1 peptides. For 8/10 of the SARS-CoV-2-derived-HLA-DR peptides selected for the CoVac-1 vaccine T-cell responses in up to 44% of unexposed donors were detected. This is most likely due to T-cell cross-reactivity based on sequence homology of SARS-CoV-2 to common cold corona viruses or other pathogens. This cross-reactivity is a well-known frequently occurring effect in viral and other pathogen T-cell immunity providing so called heterologous immunity. Heterologous immunity is defined as an immunity that can develop to one pathogen after exposure or vaccination to non-identical pathogens. Therefore, the CoVac-1 vaccine will not only induce de novo SARS-CoV-2-specific T-cell responses but further will allow to expand low frequent SARS-CoV-2-detecting T-cell populations preexisting in study volunteers/patients.

Exemplarily, results of an IFNγ ELISPOT and flow cytometry-based intracellular cytokine staining detecting naturally-induced CD4⁺ T-cell responses are displayed in Figure 2.

**Table 2: Naturally-induced T-cell responses to CoVac-1 peptides in IFNγ ELISPOT and ICS in patients recovered from SARS-CoV-2 infection and unexposed donors**

| **Sequence** | **SEQ ID NO** | **Protein** | **Protein** | **protein class** | **Start position** | **SARS cohort** | | | **PRE cohort** | |
|---|---|---|---|---|---|---|---|---|---|---|
| | | | | | | **pos/ tested** | **frequency [%]** | **ICS** | **pos/ tested** | **frequency [%]** |
| KDGIIWVATEGALNT | 1 | ORF9 | nucleocapid protein | structural | 127 | 20/22 | 91% | CD4⁺ IFNy⁺ TNF⁺ CD107a | 8/18 | 44% |
| RWYFYYLGTGPEAGL | 2 | ORF9 | nucleocapid protein | structural | 107 | 16/22 | 73% | CD4⁺ IFNy⁺ TNF⁺ CD107a | 1/18 | 6% |
| ASWFTALTQHGKEDL | 3 | ORF9 | nucleocapid protein | structural | 50 | 13/22 | 59% | CD4+ IFNy⁺ TNF⁺ | 1/18 | 6% |
| LLLLDRLNQLESKMS | 4 | ORF9 | nucleocapid protein | structural | 221 | 14/22 | 64% | CD8⁺ and CD4⁺ IFNy⁺ TNF⁺ CD107a | 1/17 | 6% |
| ASAFFGMSRIGMEVT | 5 | ORF9 | nucleocapid protein | structural | 311 | 12/23 | 52% | CD4+ IFNy⁺ TNF⁺ | 1/18 | 6% |
| VADYSVLYNSASFST | 6 | ORF2 | spike protein | structural | 362 | n.t. | | | Induction of peptide-specific CD4⁺ T-cell responses in a healthy volunteer by peptide vaccination | |
| ITRFQTLLALHRSYL | 7 | ORF2 | spike protein | structural | 235 | 12/22 | 55% | IFNy⁺ TNF⁺ CD107a⁺ | 1/20 | 5% |
| FYVYSRVKNLNSSRV | 9 | ORF4 | envelope protein | structural | 56 | 11/22 | 50% | IFNy⁺ TNF⁺ | 2/19 | 11% |
| LSYYKLGASQRVAGD | 8 | ORF5 | membrane protein | structural | 176 | 21/22 | 95% | 10x IFNy⁺ TNF⁺ 5x CD107a⁺ | 0/19 | 0% |
| SKWYIRVGARKSAPL | 10 | ORF8 | n.a. | accessory | 43 | 15/22 | 68% | 5x IFNy⁺ TNF⁺ | 4/16 | 25% |

### Representation of SARS-CoV-2-derived peptides in other species

In contrast to self-peptides used for peptide vaccination in malignancy that may be expressed at considerable levels also on normal organs virus-derived peptides do not bear the risk of inducing autoimmunity. This is supported by blast results of the peptide sequences of the CoVac-1 vaccine using the "Basic Local Alignment Search Tool (BLAST)" of the uniprot database (https://www.uniprot.org/blast/) showing for none of the peptides sequence similarities to any human protein/peptide. Most common sequence similarities were identified to SARS-CoV-1 as well as to Bat SARS-like coronavirus (Table 3).

**Table 3: Results of Basic Local Alignment Search Tool (BLAST)" of the uniprot database (https://www.uniprot.org/blast/) for sequence similarities of CoVac-1 peptide**

| **Sequence** | **SEQ ID NO** | **Organism** | **Protein name** | **Uniprot ID Protein** | **Identity [%]** | **Status** |
|---|---|---|---|---|---|---|
| KDGIIWVATEGALNT | 1 | Bat coronavirus BM48-31/BGR/ | Nucleoprotein | EOXJ00 | 93.3 | unreviewed |
| RWYFYYLGTGPEAGL | 2 | Human SARS coronavirus | Nucleoprotein | P59595 | 93.3 | reviewed |
| ASWFTALTQHGKEDL | 3 | Human SARS coronavirus | Nucleoprotein | P59595 | 93.3 | reviewed |
| LLLLDRLNQLESKMS | 4 | Human SARS coronavirus | Nucleoprotein | P59595 | 93.3 | reviewed |
| ASAFFGMSRIGMEVT | 5 | Human SARS coronavirus | Nucleoprotein | P59595 | 100.0 | reviewed |
| VADYSVLYNSASFST | 6 | Bat coronavirus HKU3 | Spike glycoprotein | Q3LZX1 | 86.7 | reviewed |
| ITRFQTLLALHRSYL | 7 | Gluconobacter wancherniae NB, Leptospira gomenensis | Bifunctional enzyme IspD/IspF, Insulinase family protein | A0A511AZ17, A0A5F1Z381 | 90 | unreviewed |
| FYVYSRVKNLNSSRV | 9 | Human SARS coronavirus | Envelope small membrane protein | P59637 | 100 | reviewed |
| LSYYKLGASQRVAGD | 8 | Bat SARS-like coronavirus | Membrane protein | A0A2R3SUX3 | 100 | unreviewed |
| SKWYIRVGARKSAPL | 10 | Fasciola hepatica | Ryanodine receptor 44F | A0A4E0RMD6 | 50.0 | unreviewed |

### Embedded HLA class I binding warehouse peptides

6/10 vaccine peptides in the CoVac-1 vaccine include embedded CD8⁺ T-cell epitopes predicted to bind to various different HLA class I allotypes. These embedded HLA class I peptides will allow to additionally activate CD8⁺ T-cells that should also contribute to prevent severe courses of COVID-19 disease. The embedded CD8⁺ T-cell epitopes are based on the inventors' preclinical work predicting and characterizing SARS-CoV-2-derived HLA class I T-cell epitopes. Therefore, the protein sequences of all ten SARS-CoV-2 ORFs were split into 9 - 12 amino acid long peptides covering the complete proteome of the virus. The prediction algorithms NetMHCpan 4.0 and SYFPEITHI 1.0 (Rammensee et al. 1999, *I.c*.) were used to predict the binding of the peptides to HLA-A*01:01, -A*02:01, -A*03:01, -A*11:01, -A*24:02, -B*07:02, -B*08:01, -B*15:01, -B*40:01, and -C*07:02. With this selection of allotypes 91.7% of the world population is covered with at least one HLA allotype. Out of the total amount of 38,424 possible 9-12mer long peptides derived from all 10 ORFs 1,739 unique HLA-binding peptides excluding peptides containing cysteine were predicted as potential SARS-CoV-2 epitopes; Nelde et al. (*I.c.*)*.* Table 4 shows the embedded CD8⁺ T-cell epitopes for each CoVac-1 peptide.

**Table 4: Embedded HLA-class I T-cell epitopes in the CoVac-1 vaccine peptides**

| **Sequence** | **SEQ ID NO** | **HLA restriction** | **embedded HLA class I peptides** |
|---|---|---|---|
| KDGIIWVATEGALNT | 1 | DRB1*01, DRB1*04, DRB1*11 | no |
| RWYFYYLGTGPEAGL | 2 | DRB1*04 | YYLGTGPEAGL (A*24) (SEQ ID NO: 11) |
| | | | YLGTGPEAGL (A*02) (SEQ ID NO: 12) |
| ASWFTALTQHGKEDL | 3 | DRB1*04, DRB1*11 | FTALTQHGK (A*03, A*11) (SEQ ID NO: 13) |
| LLLLDRLNQLESKMS | 4 | DRB1*04, DRB1*15 | LLLLDRLNQL (A*02) (SEQ ID NO: 14) |
| | | | LLLDRLNQL (A*02) (SEQ ID NO: 15) |
| ASAFFGMSRIGMEVT | 5 | DRB1*01, DRB1*04, DRB1*07, DRB1*11 | ASAFFGMSR (A*11) (SEQ ID NO: 16) |
| VADYSVLYNSASFST | 6 | DRB1*01, DRB1*04, DRB1*11 | no |
| ITRFQTLLALHRSYL | 7 | DRB1*01 | TRFQTLLAL (C*07) (SEQ ID NO: 17) |
| | | | TLLALHRSY (B*15) (SEQ ID NO: 18) |
| | | | LLALHRSYL (A*02) (SEQ ID NO: 19) |
| FYVYSRVKNLNSSRV | 9 | DRB1*04, DRB1*11 | FYVYSRVKNL (A*24) (SEQ ID NO: 20) |
| | | | RVKNLNSSR (A*03), (SEQ ID NO: 21). |
| LSYYKLGASQRVAGD | 8 | DRB1*04, DRB1*07 | no |
| SKWYIRVGARKSAPL | 10 | DRB1*01, DRB1*11 | no |

### 3D-structere analysis of SARS-CoV-2-derived peptides

Development of antibody-dependent enhancement (ADE) antibodies has been identified as potential risk for infected patients after vaccination approaches; see Graham BS. Rapid COVID-19 vaccine development. Science. 2020. In contrast to other classical vaccines aiming to induce an antibody-response to prevent viral infections the CoVac-1 vaccine is designed to induce SARS-CoV-2 specific T-cells. To further minimize the risk of ADE antibody development to the CoVac-1 vaccine peptides especially for peptides from the virus surface proteins, only sequences were selected that do not represent antibody epitopes, since they are not accessible to antibodies due to the predicted 3D structure of the protein or based on their intravirion location (Table 5). In a first *in vivo* application of a CoVac-1 vaccine peptide from the spike protein (VADYSVLYNSASFST; SEQ ID NO: 6) in a healthy volunteer no antibody induction was observed so far. Therefore, there is compared to other vaccine approaches only minimal risk of induction of ADE antibodies by the CoVac-1 vaccine.

**Table 5: Results of 3D structural analysis of CoVac-1 peptides derived from virus surface proteins**

| **Sequence** | **SEQ ID NO** | **Protein** | **Protein** | **protein class** | **exposure to antibodies** | |
|---|---|---|---|---|---|---|
| | | | | | **close state** | **open state** |
| VADYSVLYNSASFST | 6 | ORF2 | spike protein | structural | likely to be buried | exposed in a cleft |
| ITRFQTLLALHRSYL | 7 | ORF2 | spike protein | structural | RSYL unresolved, most are buried (SEQ ID NO: 22) | ALHRSYL unresolved, most are buried (SEQ ID NO: 23) |
| FYVYSRVKNLNSSRV | 9 | ORF4 | envelope protein | structural | intravirion position | |
| LSYYKLGASQRVAGD | 8 | ORF5 | membrane protein | structural | intravirion position | |

### 2. Clinical studies

### Summary

### Definition of terms

- Drug substances:: Ten SARS-CoV-2-derived HLA-DR peptides and the TLR1/2 ligand XS15
- Peptide cocktail:: Peptide cocktail for each study volunteer/patient including 10 immunogenic HLA-DR SARS-CoV-2-derived peptides and the TLR1/2 ligand XS15
- IMP/Drug Product/peptide vaccine:: CoVac-1: Peptide cocktail emulsified in Montanide ISA 51 VG
- IMP administration:: subcutaneous injection with 2ml syringe (e.g. BD Emerald) and needle (e.g. BD Eclipse Needle 27Gx1/2)

### 2.1 SARS-CoV-2-derived peptides

Each volunteer/patient enrolled in the P/T-pVAC-SARS-CoV-2 trials receive 10 promiscuous HLA-DR peptides (240 µg each) derived from different proteins of SARS-CoV-2 together with the TLR1/2 ligand XS15. For application the vaccine cocktail is emulsified in Montanide ISA 51 VG. Details on drug substances can be found in Table 1.

### Synthesis and structure

All drug substances are linear peptides with a free amino group at the N-terminus and a free carboxyl group at the C-terminus. Trifluoroacetate anions are bound in ionic form to basic groups of the peptide molecule. All amino acid residues are in the L-configuration and are not chemically modified at any positions. All warehouse peptides are manufactured in a facility holding certificates for the production of GMP grade synthetic peptides. All peptides are synthetic peptides, which are manufactured by well-established solid phase peptide synthesis (SPPS) procedures using Fmoc chemistry.

### 2.2 TLR1/2 ligand XS15

The TLR1/2 ligand XS15 is used as adjuvant in the P/T-pVAC-SARS-CoV-2 trials to induce strong and long-lasting T-cell responses. The lipopeptide XS15, chemical name N-Palmitoyl-S-[2,3-bis(palmitoyloxy)-(2R)-propyl]-(R)-cysteinyl-GDPKHPKSF, is a water-soluble synthetic Pam3Cys-derivative. As TLR1/2 ligand it will be included as an adjuvant in the peptide cocktail of each volunteer/patient enrolled in the P/T-pVAC-SARS-CoV-2 trials. XS15 hydrochloride is a synthetic linear, ten amino acid peptide with a palmitoylated N-terminus. All optically active amino acids are in L-configuration. The drug substance is isolated as a hydrochloride salt. The primary structure of XS15 Hydrochloride is given by its route of synthesis and identity can be confirmed by mass spectrometry. XS15 is manufactured (Bachem AG, Bubendorf, Switzerland) as an active pharmaceutical ingredient following the relevant GMP guidelines, namely ICH Q7 and Part 2 of the EU GMP guideline. The manufacturer has been regularly and successfully inspected by the Swiss Agency for Therapeutic Products (Swissmedic) and is responsible for manufacture, testing, and release as well as storage and shipment of XS15 hydrochloride. Please refer to the IB of XS15 (1.0. 27 May 2020).

### 2.3 Montanide ISA 51 VG

Montanide is manufactured by Seppic and by the rewarding manufacturer Elaiapharm, respectively. Montanide ISA 51 VG is used as adjuvant in the P/T-pVAC-SARS-CoV-2 trials to emulsify the peptide cocktail (including SARS-CoV-2-derived peptides and XS15). Montanide ISA 51 VG is defined as a mixture of a highly purified mineral oil (Drakeol 6VR) and a surfactant (Mannide monooleate). When mixed with an aqueous phase in a 50/50 ratio, it renders a water-in-oil emulsion. This water-in-oil emulsion is used as vaccine adjuvant, in order to enhance the immune response against antigens. For the detailed pre-clinical information on Montanide ISA 51 VG it is referred to IB of Montanide ISA 51 VG (3291/GB/03/June 2019).

### 2.4 Formulation

The peptide cocktails are formulated by the University of Tübingen (Department of Immunology) including the 10 promiscuous HLA-DR peptides (240 µg each) derived from different proteins of SARS-CoV-2 and the TLR1/2 ligand XS15. The final CoVac-1 DP is a water-in-oil emulsion of the peptide cocktail as described above and Montanide ISA 51 VG. All components will be provided by the Wirkstoffpeptidlabor of the Department of Immunology in Tübingen together with a "mixing kit" allowing for the mixture of the two components by the pharmacy of the participating centers (peptide cocktail, Montanide ISA 51 VG). The Wirkstoffpeptidlabor holds certificates for the production of GMP grade synthetic peptides and for the formulation of multi-peptide vaccine cocktails including the TLR1/2 ligand XS15. The peptide cocktails are liquid formulations consisting of the Drug Substances dissolved in 33% DMSO in water for injection (WFI). No excipients are added. Peptide cocktails are packaged in 1 ml vials using the Crystal^{®} closed vial technology and capped by a flip-off crimping cap. Each vial is for single use only. Peptide cocktails are manufactured aseptically. Analyses for endotoxins, sterility, visible particles and pH of solution are performed according to European Pharmacopeia (EP). Peptide cocktails (including the TLR1/2 ligand XS15) are labeled with an identification code definitely assignable to the clinical trial. The trial medication is labeled according to § 5 of GCP-V. Samples of the labels are filed in the trial master file (TMF). The peptide vaccine cocktail is packaged together with Montanide ISA 51 VG and the mixing equipment into the "mixing kit" and shipped from the Wirkstoffpeptidlabor of the Department of Immunology, Tübingen, to the pharmacy of the participating centers. Shipment is documented according to standard operation procedure. The "mixing kit" is shipped using isolated packaging with an automated temperature control system, which has to be returned to the Wirkstoffpeptidlabor of the Department of Immunology together with the acknowledgement of receipt after delivery of the consignment. The device is read out to document the correct storage temperatures during shipment. Data are documented according to standard operation procedure. The shipment is performed by an associate of the Wirkstoffpeptidlabor of the Department of Immunology, Tübingen. Peptide cocktail solutions should be stored at -20°C.

### 2.5 Dosage

### Peptide cocktail

Peptides cocktails for the CoVac-1 drug product (DP) are liquid formulations consisting of the drug substances dissolved in 33% DMSO. Peptides cocktails to be used in the P/T-pVAC-SARS-CoV-2 trials consist of 10 different peptides with 1mg/ml per peptide and the TLR1/2 ligand XS15 with 0,2mg/ml. Each vial contains 700µl.

### Montanide ISA 51 VG

Before delivery to the study site the peptide cocktail is emulgated in a water-oil emulsion 1:1 with Montanide ISA 51 VG by the pharmacy of the participating centers according to a "Mischanleitung" provided with the "mixing kit" from the Wirkstoffpeptidlabor of the Department of Immunology.

### CoVAC-1

The final CoVAC-1 drug product is delivered as a water-oil solution by the pharmacy of the participating centers to the respective trial site in a syringe with a final volume of 500µl (approx. 240µg per peptide, 50µg XS15).

### Instruction for handling and storage of CoVac-1 DP

One single dose of the CoVac-1 vaccine is applied subcutaneously (s.c.) into the abdominal skin of the study volunteer/patient. The study-specific instructions are described in the Clinical Study Protocols. Each CoVac-1 vial is labeled with a study specific code. Aseptic technique is used when preparing CoVac-1 vaccines for administration. Each vial of CoVac-1 DP is for single use only. Final vaccine drug product is stored at room temperature and administered within 24h after mixing of the components.

### 2.6 Dosage regimen and vaccination schedule and site

Per volunteer/patient one single vaccination with the CoVac-1 vaccine is applied subcutaneously. This is based on the first clinical experiences with individualized peptide vaccines combined with the TLR1/2 ligand XS15 in a healthy volunteer and cancer patients. The usage of XS15 enables to induce strong and long-lasting T-cell responses against vaccine peptides in so far 100% of vaccinated healthy volunteer (HV) and patients after one injection. These data allow reducing the number of vaccinations and the duration of vaccination phase compared to previous studies.

Previous vaccination trials were performed at peptide doses ranging from 10 to 5,000 µg per vaccination. Even though only a few of these trials included a dose finding element, there is a tendency that doses below 100 µg are not effective to induce T-cell responses whilst doses above 500 µg do not seem to generate an increasing immunogenicity. Dose-finding studies performed with viral protein-derived epitopes showed significantly stronger immune responses in the 300-500 µg range versus the 100 µg dose, without significantly higher immune responses in the 1,000 vs. 500 µg group (Kran et al. HLA- and dose-dependent immunogenicity of a peptide-based HIV-1 immunotherapy candidate (Vacc-4x). AIDS. 2004;18(14):1875-1883). Based on the observations briefly summarized above, the ideal dose for a peptide is anticipated to be in the range between 100 to 500 µg with a strong preference to the upper range. The dose of -240 µg per peptide per dose for CoVac-1 vaccine was selected based on these findings and on the feasibility in pharmaceutical development of the vaccines. With this dose induction of strong T-cell response for two of the peptides included in the CoVac-1 vaccine in combination with XS15 emulsified in Montanide ISA 51 VG could be shown.

### Rational for the CoVac-1 peptide dose

Based on the following data the peptide vaccine is applied with a dose of 240 µg per peptide:
- The phase II and III peptide vaccination trials performed by Immatics Biotechnologies GmbH in patients with metastatic RCC demonstrated a very good safety and tolerability profile for a peptide cocktail with 10 peptides and a dose of 413 µg per peptide. Another study of the Department of Immunology in Tübingen in patients with metastatic prostate cancer also showed a good tolerability for a vaccine cocktail including 13 peptides with a dose of 300 µg per peptide (Feyerabend et al. Novel multi-peptide vaccination in Hla-A2+ hormone sensitive patients with biochemical relapse of prostate cancer. Prostate. 2009;69(9):917-927).
- Sato et. al. (Sato et al. Immunological evaluation of peptide vaccination for patients with gastric cancer based on pre-existing cellular response to peptide. Cancer Sci. 2003;94(9):802-808) investigated a peptide vaccine in patients with advanced gastric cancer composed of 14-16 peptides with a dose of 30 mg per peptide. Even with that very high dose of peptide vaccine, no severe side effects were observed. Furthermore, the group of Noguchi (Noguchi et al. Induction of cellular and humoral immune responses to tumor cells and peptides in HLA-A24 positive hormone-refractory prostate cancer patients by peptide vaccination. Prostate. 2003;57(1):80-92) showed a good tolerability and safety profile for a peptide vaccine composed of 14 peptides and a dose of 9mg per peptide.
- An ongoing iVAC-CLL01 study applies personalized peptide vaccine cocktails (9 peptides a 300 µg) showing a very good safety and tolerability profile.
- Preliminary data from a healthy volunteer and cancer patients vaccinated with a personalized peptide vaccine (240-300 µg per peptide) in combination with XS15 showed potent induction of T-cell responses in 100% of healthy volunteer (HV) and patients and a good safety profile.
- Preliminary data from a healthy volunteer vaccinated with two of the peptides (240 µg per peptide) included in the CoVac-1 vaccine in combination with XS15 emulsified in Montanide ISA 51 VG showed induction of strong CD4⁺ T-cell response and a good safety profile.

### Monitoring after peptide vaccination

To provide a maximum of safety for the patients treated in the P/T-pVAC-SARS-CoV-2 studies, the monitoring after peptide vaccination is performed as follows:
- All patients are monitored for 2 hours after vaccination, including close monitoring of heart rate, blood pressure, temperature and subjective well-being. The peptide vaccination studies conducted in infectious disease as well as cancer patients showed that a monitoring time of 1-2 hours provides optimal safety for the patient. In these studies, allergic reactions occurred mostly 1 hour after vaccination.
- The monitoring of the patients is carried out by specially trained nurses and physicians. Monitoring includes regular checks of the vital parameters of the patient (blood pressure, pulse, temperature, oxygen saturation) to ensure early notice of side effects, especially allergic reactions. Each monitoring unit is equipped with a crash cart and an intensive care team will be on standby.

### Vaccination site

The CoVac-1 vaccines are applied subcutaneously in the abdomen of the patients based on the following rational:

The peptide vaccine IMA901 (for treatment of RCC) has been tested in two completed clinical studies where it had been applied to a total of 96 patients. In the phase I study (n=30), vaccination to the thigh, abdomen and skin of the forearm were allowed. In 27 patients evaluable for immune responses, vaccination in the skin of the abdomen was numerically, but not significantly better in terms of achieved immune response rate (88% n=8 vs. 64% n=14, and 60% n=5) for thigh and forearm, respectively; e.g. p=0.75 abdomen vs. other; Fisher's exact test). In the phase II study (n=68), vaccinations to the thigh and abdomen were allowed, and again immune responder rates and multi-peptide responder rates were very comparable and did not differ significantly from each other (e.g. 27.5% multi-peptide responders after vaccination into the abdominal skin (n=40) vs. 23.8% after vaccination into the thigh (n=21). In addition, preclinical work in a mouse model (brain tumor, glioma) showed that vaccination into the foreleg is less effective in inducing immune responses as compared to vaccination into a more distant site (hind leg), potentially due to the immunosuppressive influence of the tumor in the tumor draining lymph (Ohlfest et al. Vaccine injection site matters: qualitative and quantitative defects in CD8 T-cells primed as a function of proximity to the tumor in a murine glioma model. J Immunol. 2013;190(2):613-620).

### 2.7 Preclinical information

### SARS-CoV-2-derived peptides (drug substances)

The peptide cocktail included in the CoVac-1 vaccine consists of ten promiscuous HLA-DR peptides from different proteins (ORFs) of the SARS-CoV-2 virus. Using the algorithm SYFPEITHI (www.syfpeithi.de; Rammensee et al. 1999, *I.c*.) peptide sequences were selected that show a high binding score for several HLA-DR allotypes and can therefore be widely used in the population. Furthermore, especially those HLA-DR peptides were selected that contain embedded HLA class I sequences in order to induce CD4⁺ T-cell responses as well as CD8⁺ T-cell responses. Furthermore, for peptides from virus surface proteins, only sequences were selected that do not represent antibody epitopes, since they are not accessible to antibodies due to the predicted 3D structure of the protein. This should prevent the formation of antibodies against the vaccinated peptides, which could possibly lead to a deterioration of the lung pathology. Immunogenicity was proven for all HLA-DR peptides included in the peptide cocktail in a large cohort of SARS-CoV-2 convalescent donors proving the significant role of these T-cell epitopes in the natural course of SARS-CoV-2 infection. Furthermore, two peptides were already applied in a vaccination approach in a single healthy volunteer, inducing strong CD4⁺ T-cell responses. The SARS-CoV-2-derived HLA-DR peptides will activate CD4⁺Th1 cells which directly contribute to virus clearance and deliver strong T-helper signals to the CD8⁺ T-cells primed by embedded HLA class I sequences in the CoVac-1 vaccine or during natural infection. Furthermore, in terms of a SARS-CoV-2 infection these SARS-CoV-2 specific CD4+Th1 cells should vigorously activate virus antigen-experienced B cells. The resulting enhanced activity could lead to more rapid virus clearance and prevention of severe course of COVID-19.

### 2.8 Pharmacokinetics

The CoVac-1 vaccine is injected subcutaneously. Biodistribution and pharmacokinetic studies for the included SARS-CoV-2-derived peptides have not been performed as they are usually not conducted on vaccines since the vaccine components are intended to immediately bind to HLA molecules expressed on local cell populations (i.e. dendritic cells (DCs)). In addition, peptides are generally extremely short-lived in biological fluids.

### 2.9 Clinical experience

Peptide vaccines in combination with XS15 emulsified in Montanide ISA 51 VG similar to the CoVaC-1 DP have been applied in a healthy volunteer (Rammensee et al. A new synthetic toll-like receptor 1/2 ligand is an efficient adjuvant for peptide vaccination in a human volunteer. J Immunother Cancer. 2019;7(1):307) and cancer patients inducing a strong and long lasting CD8⁺ and Th1CD4⁺ T-cell response to viral peptides (including SARS-CoV-2 derived peptides), neoepitopes from cancer-specific mutations as well as to tumor-associated self-peptides. Beside formation of granuloma locally on injection side, no relevant side effects, especially no allergic or anaphylactic reactions or cytokine release syndrome, have been observed after a single application of the vaccine. Montanide ISA 51 VG has been used in more than 200 clinical trials including more than 6000 patients. Most common side effects are injection site reaction (68%) including granuloma development, fatigue (54%), fever (41%), gastrointestinal disorders (32%) and injection site or local erythema (28%) (Van Doorn et al. Safety and tolerability evaluation of the use of Montanide ISA51 as vaccine adjuvant: A systematic review. Hum Vaccin Immunother. 2016;12(1):159-169). In general, the observed adverse from controlled trials with non-healthy as well as healthy individuals were mild to moderate in intensity. For more details it is referred to the IB of Montanide ISA 51 VG (3291/GB/03/June 2019).

### 2.10 Choice of adjuvant drugs for the P/T-pVAC-SARS-CoV-2 study

For the P/T-pVAC-SARS-CoV-2 studies the following considerations on adjuvant drugs were made:

A main prerequisite for peptide vaccination, beside the selection of optimal antigen targets, is the usage of a suitable adjuvant, which is able to induce strong and long-lasting immune responses. Among the most effective peptide vaccination methods tested in humans is the subcutaneous injection of peptides emulsified in Montanide ISA 51 VG, a water-in-oil-emulsion, combined with the TLR9 ligand CpG (Baumgaertner et al. Vaccination-induced functional competence of circulating human tumor-specific CD8 T-cells. Int J Cancer. 2012;130(11):2607-2617). However, CpG is not available for clinical trials and a peptide/antigen vaccine emulsified in Montanide without any additional adjuvant shows only week immune response (Freund J. The effect of paraffin oil and mycobacteria on antibody formation and sensitization; a review. Am J Clin Pathol. 1951;21(7):645-656.). Therefore, the inventors choose for the P/T-pVAC-SARS-CoV-2 study the novel TLR1/2 ligand XS15. XS15 is a water-soluble derivative of the TLR1/2 ligand Pam3Cys inducing a strong CD8+ and Th1CD4⁺ T-cell response against free short peptides in Montanide ISA 51 VG after s.c. injection in healthy volunteers as well as in patients (Rammensee et al. A new synthetic toll-like receptor 1/2 ligand is an efficient adjuvant for peptide vaccination in a human volunteer. J Immunother Cancer. 2019;7(1):307). Immune responses could be induced for viral peptides (including SARS-CoV-2-derived peptides), neoepitopes from cancer-specific mutations as well as for tumor-associated self-peptides. Peptide vaccination with XS15 emulsified in Montanide ISA 51 VG results in granuloma formation on vaccination site where the vaccinated peptides persist for at least 7 weeks. Furthermore peptide-specific T-cells could also be detected on granuloma site, however with a lower frequency than observed in peripheral blood, which confirms that there is no risk of T-cell sequestration, dysfunction or deletion on vaccination site using XS15 in Montanide ISA 51 VG. Strikingly, the induced immune responses persist for more than 1.5 years. Multi-peptide vaccination with XS15 emulsified in Montanide ISA 51 VG showed a good safety profile without any systemic side effects so far no allergic reactions as it was reported for peptide vaccines applied in combination with GM-CSF. Therefore, XS15 and Montanide ISA 51 VG will serve as adjuvants for the CoVac-1 vaccines. Detailed information to the lipopeptide XS15 which is included in the vaccine cocktail can be found in the IB of XS15 (1.0. 27 May 2020). Detailed information to Montanide ISA 51 VG can be found in the IB of Montanide ISA 51 VG (3291/GB/03/June 2019).

### 2.11 Effects of XS15 in humans

### Evidence from self-administration of XS15 adjuvanted vaccines

Vaccination of a healthy individual using XS15 as an adjuvant has been described and published (Rammensee et al. 2019, I.c.) In this self-experiment a dosage of 80 µg XS15 was used in addition to Montanide ISA 51 VG and a multi-peptide vaccine with virus-derived synthetic peptides. Here a painless granuloma forming at the injection site was described with a volume increasing to about 8 ml, as measured by ultrasound (days 17 and 41 after administration), without any sonographic signs of infection. After 21 days it appeared as a well-palpable induration of about 2 × 2 cm, with a central reddish surface. The granuloma was described as not touch-sensitive, whereas the skin surface reported to be slightly itching.

Regarding immune responses and being aware that this is purely anecdotal evidence, a single vaccination induced strong *ex vivo* measurable CD8⁺ and TH1 CD4⁺ responses in a human volunteer upon a single injection of XS15 mixed to uncoupled peptides in a water-in-oil emulsion. The granuloma that formed locally at the injection site containing highly activated functional CD4⁺ and CD8⁺ effector memory T-cells. In addition, respective T-cells could be characterized in circulation. *Ex vivo* T-cell responses in peripheral blood were detectable for more than one year and could be strongly boosted by a second vaccination.

A more current report of self-vaccination of the same individual includes the administration of SARS-CoV-2-derived peptides performed in March 2020. Here, the self-experimenting healthy volunteer used eight SARS-CoV-2-derived peptides: five predicted to bind to HLA class I molecules (CD8 peptides) and three predicted to bind to HLA-DR molecules (CD4 peptides, including two peptides from the CoVac-1 vaccine). The vaccine formulation also included one long and one short CMV-pp65-derived peptide that had previously been administered to the same individual and could thus act as positive controls. About 3 weeks after a single vaccination, using a vaccine containing the mentioned synthetic peptides, Montanide and containing 50 µg XS15, strong vaccine-induced CD4⁺ T-cell responses against all four HLA-DR peptides and against the recall CMV epitope could be detected. No T-cell responses against the five predicted SARS-CoV-2 HLA class I peptides could be detected by ex vivo Interferon-γ ELISpot assay. Furthermore no antibody induction against the vaccine peptides was observed83. Vaccination was well tolerated without any systemic side effects. Granuloma formation was observed at the vaccination site.

### Preliminary evidence from unproven interventions using XS15-adjuvanted vaccines in cancer patients

In unproven clinical interventions using XS15-adjuvanted vaccines as a treatment effort according to physician's judgement and with informed consent, in keeping with the principle 37 of the Declaration of Helsinki, cancer patients with different malignancies were vaccinated. Respective patients had diverse pre-treatments and have been vaccinated with personalized peptide vaccines in combination with XS15 (50 µg) emulsified in Montanide ISA 51 VG. A detailed description of the patient's characteristics is provided below (Table 6). Each peptide vaccine was designed individually for every patient, based on MS/MS-based HLA ligandom analyses and panel sequencing characterizing individual mutations in the tumor tissue for target discovery. Up to three vaccinations were administered per patient. The mean follow-up period per patient is 14 months (2-36 months) following the first vaccination. The described vaccines were applied s.c. to the abdominal skin. All vaccinated patients showed peptide vaccine-specific T-cell responses after the first vaccination, which could be boosted by subsequent vaccinations. Induced T-cell responses were long-lasting (up to 19 month) after the end of vaccination (Figure 3).

Due to the anecdotal character of the interventions described here such as the major differences between patients as well as diverse cancer entities, pre-treatment, vaccine composition and concomitant (cancer) medication, an joint analysis of the data regarding these peptide vaccinations in combination with XS15 is not intended. The characteristics of subjects receiving individualized peptide vaccines in combination with XS15 are provided below.

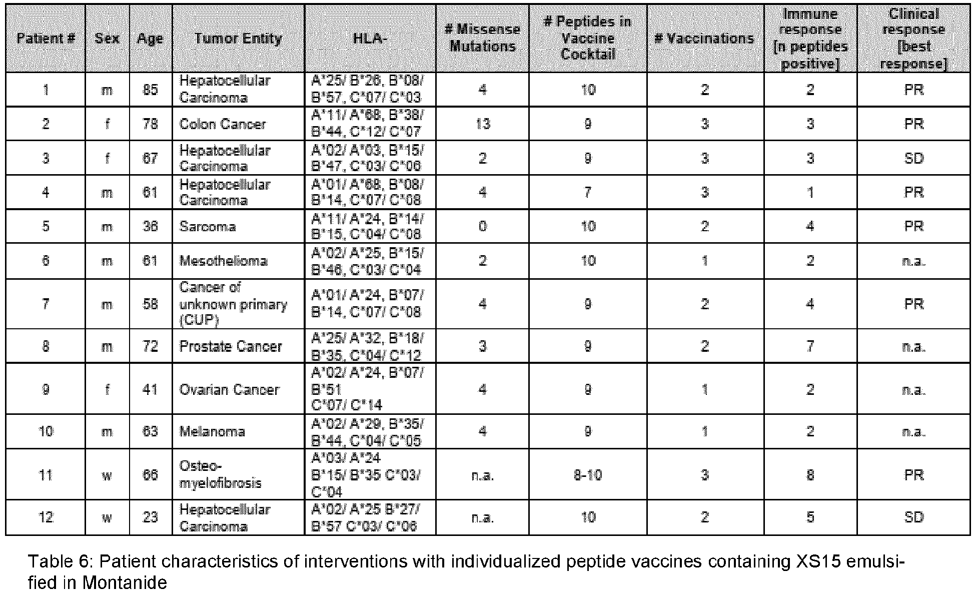

### Adverse events profile

No systemic adverse events occurred in any of the vaccinated patients and the vaccinations were well-tolerated. No fever or any other sign of exuberant immune reaction was observed. In accordance with the expectable adverse events profile of Montanide an asymptomatic subcutaneous granuloma evolved over 1-3 weeks after vaccination. The granulomas usually persist for up to eight weeks in individual patients. Most common related events were transient redness and swelling at the vaccination side (Table 7). After multiple vaccination granuloma ulceration was observed In 5/12 patients (41%), resulting in an aseptic skin ulceration that healed within two weeks not requiring any further intervention or treatment.

**Table 7: Summary of observed local events attributed to Montanide use in cancer patients.**

| **Patient #** | **Local reaction at vaccination side** | |
|---|---|---|
| | **After 1. vaccination** | **After multiple vaccinations** |
| 1 | asymptomatic s.c. granuloma (max. ∅ 0.5 cm) | asymptomatic s.c. granuloma (max. ∅ 2 cm) |
| 2 | asymptomatic s.c. granuloma (max. ∅ 3 cm) | asymptomatic s.c. granuloma (max. ∅ 3 cm) |
| 3 | s.c. granuloma (max. ∅ 2 cm), redness and swelling of the skin | s.c. granuloma (max. ∅ 2 cm), redness and swelling of the skin, aseptic skin ulceration |
| 4 | asymptomatic s.c. granuloma (max. ∅ 3 cm) | s.c. granuloma (max. ∅ 4 cm), redness and swelling of the skin, aseptic skin ulceration |
| 5 | asymptomatic s.c. granuloma (max. ∅ 1 cm) | s.c. granuloma (max. ∅ 2 cm), redness and swelling of the skin, aseptic skin ulceration |
| 6 | asymptomatic s.c. granuloma (max. ∅ 1 cm) | asymptomatic s.c. granuloma (max. ∅ 2 cm) |
| 7 | asymptomatic s.c. granuloma (max. ∅ 1 cm) | s.c. granuloma (max. ∅ 3 cm), redness and swelling of the skin |
| 8 | s.c. granuloma (max. ∅ 3 cm), redness and swelling of the skin | s.c. granuloma (max. ∅ 3 cm), redness and swelling of the skin, aseptic skin ulceration |
| 9 | asymptomatic s.c. granuloma (max. ∅ 2 cm) | s.c. granuloma (max. ∅ 2 cm), redness and swelling of the skin |
| 10 | asymptomatic s.c. granuloma (max. ∅ 1 cm) | asymptomatic s.c. granuloma (max. ∅ 1 cm) |
| 11 | s.c. granuloma (max. ∅ 2 cm), redness and swelling of the skin | s.c. granuloma (max. ∅ 4 cm), redness and swelling of the skin, aseptic skin ulceration |
| 12 | asymptomatic s.c. granuloma (max. ∅ 2 cm) | asymptomatic s.c. granuloma (max. ∅ 3 cm) |

### 3. Benefits

The clinical benefits of CoVac-1 vaccination are based on the following aspects:
* Peptide vaccination using HLA-presented peptides represents an established immunotherapy approach for preventive vaccine development in infectious disease as well as for therapeutic approaches in malignant disease. Several therapeutic peptide vaccination studies in patients with malignant disease including solid tumors and hematological malignancies90-93 have proven safety and tolerability of this approach.
* Therapeutic multi-peptide vaccination represents a low side-effect immunotherapy approach relying on specific immune recognition of HLA-presented peptides.
* All peptides included in the CoVac-1 vaccine are proven SARS-CoV-2 T-cell epitopes with pathophysiological relevance in the natural course of COVID-19 disease
* CoVac-1 peptide vaccination is expected to induce potent CD8⁺ and CD4⁺Th1 T-cell responses against SARS-CoV-2, resulting in immunity against infection as:
   - CD4⁺Th1 cells directly contribute to virus clearance and deliver strong T helper signals to CD8⁺ T-cells primed during natural infection. Furthermore, such SARS-CoV-2 specific CD4⁺Th1 cells activate virus antigen-experienced B cells. The resulting increase in activity is expected to allow for rapid virus clearance and prevention of severe course of COVID-19 disease.
   - The peptides used for CoVac-1 vaccine contain embedded CD8⁺ T-cell epitopes predicted to bind to many different HLA class I allotypes. Once activated, the CD8⁺ T-cells may contribute to faster virus clearance.
* Participant selection is based on medical imperative and safety considerations:
   - The trial compromises three parts (cohorts of participants) with different age groups to provide preliminary results on safety in a young (18-55 years, n= 12) patient cohort, which is then extended to older (Part II and Part III) participants. Of note, the risk of vaccine related (S)AEs is hypothesized to be similar in each age group. By this design, the inventors address the urgent medical need for protection of subjects at risk for severe SARS-CoV-2 infection by providing safety and immunogenicity data as well as first efficacy data on the course of SARS-CoV-2 infection in this population.
   - Safety is continuously monitored by an independent data and safety monitoring board (DSMB), which will be provided with reports on frequent basis.

The clinical benefits of peptide vaccination in combination with the TLR1/2 ligand XS15 in Montanide ISA 51 VG are based on the following aspects:
* Peptide vaccination alone is rarely able to induce clinically effective T-cell responses; thus, the peptide vaccine has to be combined with an adjuvant drug to enhance immune responses.
* Several Toll-like-receptor (TLR) ligands have shown to induce strong CD8⁺/ CD4⁺Th1 responses in humans including CPG (TLR9 ligand), imiquimod (TLR7 ligand) and poly-IC (TLR3 ligand). However, for none of these TLR ligands GMP substance is available for adding to a peptide vaccine.
* XS15 is a water-soluble derivative of the TLR2 ligand Pam3Cys and induces a strong CD8⁺ and CD4⁺Th1 T-cell response against free short peptides emulsified in Montanide ISA 51 VG after a single s.c. injection in healthy volunteers as well as in patients.
* Using XS15, immune responses could be induced for viral peptides including SARS-CoV-2 specific peptides, neoepitopes from cancer-specific mutations as well as for tumor-associated self-peptides.
* XS15 results in granuloma formation on the vaccination site, where the vaccinated peptides persist for at least 7 weeks, which might support the induction of a strong immune response.
* The induced immune responses observed so far persisted for more than 1.5 years.
* Peptide vaccination in combination with XS15 in Montanide ISA 51 VG has already been applied in a healthy volunteer and cancer patients. Beside formation of granuloma locally on injection side, no relevant side effects, especially no allergic or anaphylactic reactions or cytokine release syndrome, have been observed.
* Montanide ISA 51 VG is an oil adjuvant suitable for human injection that allows the manufacturing of water in oil emulsions. Montanide ISA 51 VG has been used in more than 200 clinical trials including more than 6000 patients and was proven to be well tolerated.

## Claims

1. A peptide comprising an amino acid sequence selected from the group consisting of SEQ ID NO: 1 to SEQ ID NO: 10, and variant sequences thereof which are at least 88% homologous to SEQ ID NO: 1 to SEQ ID NO: 10, and wherein said variant binds to molecule(s) of the major histocompatibility complex (MHC) and/or induces T-cells cross-reacting with said variant peptide; and a pharmaceutical acceptable salt thereof, wherein said peptide is not a full-length polypeptide.

2. The peptide according to claim 1, wherein said peptide has the ability to bind to an MHC class-I or-II molecule, and wherein said peptide, when bound to said MHC, is capable of being recognized by CD4 and/or CD8 T-cells, preferably the amino acid sequence of said peptide comprises a continuous stretch of amino acids according to any one of SEQ ID NO: 1 to SEQ ID NO: 10.

3. An antibody, in particular a soluble or membrane-bound antibody, preferably a monoclonal antibody or fragment thereof, that specifically recognizes the peptide or variant thereof according to claim 1 or 2, preferably the peptide or variant thereof according to claims 1 or 2 when bound to an MHC molecule.

4. A T-cell receptor, preferably soluble or membrane-bound, or a fragment thereof, that is reactive with an HLA ligand, wherein said ligand is the peptide or variant thereof according to claim 1 or 2, preferably the peptide or variant thereof according to claim 1 or 2 when bound to an MHC molecule.

5. A nucleic acid, encoding for a peptide or variant thereof according to claim 1 or 2, an antibody or fragment thereof according to claim 3, a T-cell receptor or fragment thereof according to claim 4, optionally linked to a heterologous promoter sequence, or an expression vector expressing said nucleic acid.

6. A recombinant host cell comprising the peptide according to claim 1 or 2, the antibody or fragment thereof according to claim 3, the T-cell receptor or fragment thereof according to claim 4 or the nucleic acid or the expression vector according to claim 5, wherein said host cell preferably is selected from an antigen presenting cell, such as a dendritic cell, a T-cell or an NK cell.

7. An *in vitro* method for producing activated T lymphocytes, the method comprising contacting *in vitro* T-cells with antigen loaded human class I or II MHC molecules expressed on the surface of a suitable antigen-presenting cell or an artificial construct mimicking an antigen-presenting cell for a period of time sufficient to activate said T-cells in an antigen specific manner, wherein said antigen is a peptide according to claim 1 or 2.

8. An activated T lymphocyte, produced by the method according to claim 7, which selectively recognizes a cell which presents a polypeptide comprising an amino acid sequence given in claim 1 or 2.

9. A pharmaceutical composition comprising at least one active ingredient selected from the group consisting of the peptide according to claim 1 or 2, the antibody or fragment thereof according to claim 3, the T-cell receptor or fragment thereof according to claim 4, the nucleic acid or the expression vector according to claim 5, the recombinant host cell according to claim 6, or the activated T lymphocyte according to claim 7, or a conjugated or labelled active ingredient, and a pharmaceutically acceptable carrier, and optionally, pharmaceutically acceptable excipients and/or stabilizers,
preferably said pharmaceutical composition comprises at least 5, preferably at least 6, further preferably at least 7, further preferably at least 8, further preferably at least 9, and further preferably at least 10 different peptides, each peptide comprising an amino acid sequence selected from the group consisting of SEQ ID NO: 1 to SEQ ID NO: 10 or SEQ ID NO: 1, 2, 3, 4, 7, 8, 9, and 10, and variant sequences thereof which are at least 88% homologous to SEQ ID NO: 1 to SEQ ID NO: 10 or SEQ ID NO: 1, 2, 3, 4, 7, 8, 9, and 10, and wherein said variant binds to molecule(s) of the major histocompatibility complex (MHC) and/or induces T-cells cross-reacting with said variant peptide; and a pharmaceutical acceptable salt thereof, wherein said peptide is not a full-length polypeptide.

10. A pharmaceutical composition, preferably being a vaccine, comprising peptides **characterized by** the following amino acid sequences SEQ ID NO: 1, 2, 3, 4, 7, 8, 9, and 10:
KDGIIWVATEGALNT (SEQ ID NO: 1)
RWYFYYLGTGPEAGL (SEQ ID NO: 2)
ASWFTALTQHGKEDL (SEQ ID NO: 3)
LLLLDRLNQLESKMS (SEQ ID NO: 4)
ITRFQTLLALHRSYL (SEQ ID NO: 7)
LSYYKLGASQRVAGD (SEQ ID NO: 8)
FYVYSRVKNLNSSRV (SEQ ID NO: 9)
SKWYIRVGARKSAPL (SEQ ID NO: 10).

11. The pharmaceutical composition according to claim 9 or 10, wherein it additionally comprises an adjuvant.

12. The pharmaceutical composition according to claim 11, wherein the adjuvant is selected from the group consisting of TLR1/2 ligand, TLR1/2 ligand XS15, TLR1/2 ligand Pam3Cys, TLR9 ligand, TLR9 ligand CpG, Montanide ISA 51 VG, and combinations thereof.

13. The peptide according to claim 1 or 2, the antibody or fragment thereof according to claim 3, the T-cell receptor or fragment thereof according to claim 4, the nucleic acid or the expression vector according to claim 5, the recombinant host cell according to claim 6, or the activated T lymphocyte according to claim 8 for use in medicine; preferably the use is in diagnosis and/or prevention and/or treatment of an infection by SARS-CoV-2 (COVID-19), or for use in the manufacture of a medicament against an infection by SARS-CoV-2 (COVID-19).

14. The peptide according to claim 13, wherein said medicament is a vaccine.

15. A kit comprising:
(a) a container comprising a pharmaceutical composition containing the peptide(s) or the variant according to claim 1 or 2, the antibody or fragment thereof according to claim 3, the T-cell receptor or fragment thereof according to claim 4, the nucleic acid or the expression vector according to claim 5, the recombinant host cell according to claim 6, or the activated T lymphocyte according to claim 8, in solution or in lyophilized form;
(b) optionally, a second container containing a diluent or reconstituting solution for the lyophilized formulation;
(c) optionally, at least one more peptide selected from the group consisting of SEQ ID NO: 1 to SEQ ID NO: 10, and
(d) optionally, instructions for (i) use of the solution or (ii) reconstitution and/or use of the lyophilized formulation.


SEQUENCE LISTING

<110>  Eberhard Karls Universität, Medizinische Fakultät

<120>  Peptides and combinations of peptides for use in immunotherapy
       against an infection by SARS-CoV-2 (COVID-19)

<130>  5402P614EP

<160>  23

<170>  PatentIn version 3.5

<210>  1
<211>  15
<212>  PRT
<213>  SARS-CoV-2

<400>  1

Lys Asp Gly Ile Ile Trp Val Ala Thr Glu Gly Ala Leu Asn Thr
1               5                   10                  15


<210>  2
<211>  15
<212>  PRT
<213>  SARS-CoV-2

<400>  2

Arg Trp Tyr Phe Tyr Tyr Leu Gly Thr Gly Pro Glu Ala Gly Leu
1               5                   10                  15


<210>  3
<211>  15
<212>  PRT
<213>  SARS-CoV-2

<400>  3

Ala Ser Trp Phe Thr Ala Leu Thr Gln His Gly Lys Glu Asp Leu
1               5                   10                  15


<210>  4
<211>  15
<212>  PRT
<213>  SARS-CoV-2

<400>  4

Leu Leu Leu Leu Asp Arg Leu Asn Gln Leu Glu Ser Lys Met Ser
1               5                   10                  15


<210>  5
<211>  15
<212>  PRT
<213>  SARS-CoV-2

<400>  5

Ala Ser Ala Phe Phe Gly Met Ser Arg Ile Gly Met Glu Val Thr
1               5                   10                  15

<210>  6
<211>  15
<212>  PRT
<213>  SARS-CoV-2

<400>  6

Val Ala Asp Tyr Ser Val Leu Tyr Asn Ser Ala Ser Phe Ser Thr
1               5                   10                  15

<210>  7
<211>  15
<212>  PRT
<213>  SARS-CoV-2

<400>  7

Ile Thr Arg Phe Gln Thr Leu Leu Ala Leu His Arg Ser Tyr Leu
1               5                   10                  15

<210>  8
<211>  15
<212>  PRT
<213>  SARS-CoV-2

<400>  8

Leu Ser Tyr Tyr Lys Leu Gly Ala Ser Gln Arg Val Ala Gly Asp
1               5                   10                  15

<210>  9
<211>  15
<212>  PRT
<213>  SARS-CoV-2

<400>  9

Phe Tyr Val Tyr Ser Arg Val Lys Asn Leu Asn Ser Ser Arg Val
1               5                   10                  15

<210>  10
<211>  15
<212>  PRT
<213>  SARS-CoV-2

<400>  10

Ser Lys Trp Tyr Ile Arg Val Gly Ala Arg Lys Ser Ala Pro Leu
1               5                   10                  15

<210>  11
<211>  11
<212>  PRT

<213>  Homo sapiens

<400>  11

Tyr Tyr Leu Gly Thr Gly Pro Glu Ala Gly Leu
1               5                   10


<210>  12
<211>  10
<212>  PRT
<213>  Homo sapiens

<400>  12

Tyr Leu Gly Thr Gly Pro Glu Ala Gly Leu
1               5                   10


<210>  13
<211>  9
<212>  PRT
<213>  Homo sapiens

<400>  13

Phe Thr Ala Leu Thr Gln His Gly Lys
1               5


<210>  14
<211>  10
<212>  PRT
<213>  Homo sapiens

<400>  14

Leu Leu Leu Leu Asp Arg Leu Asn Gln Leu
1               5                   10


<210>  15
<211>  9
<212>  PRT
<213>  Homo sapiens

<400>  15

Leu Leu Leu Asp Arg Leu Asn Gln Leu
1               5


<210>  16
<211>  9
<212>  PRT
<213>  Homo sapiens

<400>  16

Ala Ser Ala Phe Phe Gly Met Ser Arg
1               5

<210>  17
<211>  9
<212>  PRT
<213>  Homo sapiens

<400>  17

Thr Arg Phe Gln Thr Leu Leu Ala Leu
1               5


<210>  18
<211>  9
<212>  PRT
<213>  Homo sapiens

<400>  18

Thr Leu Leu Ala Leu His Arg Ser Tyr
1               5


<210>  19
<211>  9
<212>  PRT
<213>  Homo sapiens

<400>  19

Leu Leu Ala Leu His Arg Ser Tyr Leu
1               5


<210>  20
<211>  10
<212>  PRT
<213>  Homo sapiens

<400>  20

Phe Tyr Val Tyr Ser Arg Val Lys Asn Leu
1               5                   10


<210>  21
<211>  9
<212>  PRT
<213>  Homo sapiens

<400>  21

Arg Val Lys Asn Leu Asn Ser Ser Arg
1               5


<210>  22
<211>  4
<212>  PRT
<213>  SARS-CoV-2

<400>  22

Arg Ser Tyr Leu

1

<210>  23
<211>  7
<212>  PRT
<213>  SARS-CoV-2

<400>  23

Ala Leu His Arg Ser Tyr Leu
1               5